# EUROPEAN PATENT APPLICATION

(11) **EP 4 837 900 A1**
(43) Date of publication of application: **11.11.2026**
(21) Application number: 24915254.7
(22) Date of filing: 31.12.2024
(51) Int. Cl.: C07D 213/76, C07D 213/73, A61K 31/44, A61K 31/16, A61P 35/00

(54) **AROMATIC HYDRAZIDE DERIVATIVE AND MEDICAL USE THEREOF**

(30) Priority: 01.01.2024 CN 202410000060
(71) Applicant: HUAJIAN FUTURE (CHENGDU) TECHNOLOGY CO., LTD., Sichuan 611130 (CN)
(72) Inventor: DU, Fengtian, Chengdu, Sichuan 611130 (CN); GUO, Na, Chengdu, Sichuan 611130 (CN); YANG, Xiangyu, Chengdu, Sichuan 611130 (CN); LUO, Shuai, Chengdu, Sichuan 611130 (CN); TIAN, Jingyu, Chengdu, Sichuan 611130 (CN); QIAN, Qingqing, Chengdu, Sichuan 611130 (CN); ZHANG, Li, Chengdu, Sichuan 611130 (CN)
(74) Representative: Kador & Partner Part mbB
(86) International application number: PCT/CN2024/144319
(87) International publication number: WO 2025/146040

(57) **Abstract**

The present disclosure belongs to the field of medicinal chemistry, and relates to an aromatic hydrazide derivative and the medical use thereof. Specifically, the present disclosure discloses an aromatic hydrazide derivative, a stereoisomer thereof, a tautomer thereof or a pharmaceutically acceptable salt thereof, and further discloses a preparation method for the compound and the use of the compound in the prevention and/or treatment of a tumor disease. The compound shows a good application prospect in the prevention and/or treatment of a MAPK pathway-related tumor.

## Description

### TECHNICAL FIELD

The present disclosure belongs to the field of medicinal chemistry, and relates to an aromatic hydrazide derivative, a preparation method therefor, and use of the compound in the manufacture of an anti-tumor medicament.

### BACKGROUD

Hydrazide is widely present in structures of physiologically active molecules and pharmaceutical molecules, has a unique structure playing an important role in improving drug properties, and has attracted extensive interest from domestic and foreign researchers in the process of drug design and discovery. For example, the following drug molecules all contain a hydrazide structural unit.

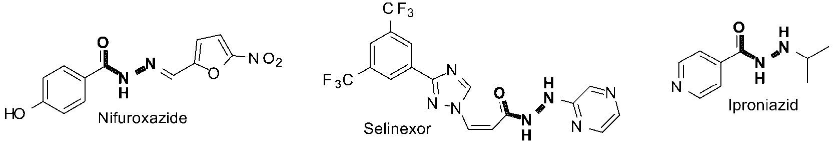

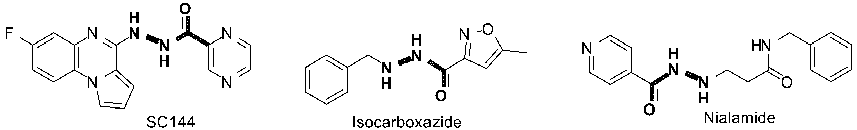

Mitogen-activated protein kinase (MAPK) belongs to a serine/threonine kinase. Extracellular signal-regulated kinase Ras/Raf/MEK/ERK signaling pathway is one of the most important cell signaling pathways, which plays a core regulatory role in many key aspects such as cell proliferation, differentiation, apoptosis and metabolism. Studies have shown that the signaling pathway is highly conserved in eukaryotic cells. When cells are stimulated by mitogens, conformational changes of Ras cause a Raf/MEK/ERK cascade reaction. Activated ERK entering the cells may allow the phosphorylation of various substrates such as kinases and transcription factors, subsequently leading to a series of physiological and biochemical reactions.

The Ras/Raf/MEK/ERK signaling pathway is one of the most important dysregulated signaling pathways in the development and progression of human tumors, which participates in various aspects such as tumor cell proliferation, apoptosis, invasion and metastasis. The pathway has become an important therapeutic target, and drugs targeting the pathway can significantly inhibit oncogenic signals in tumor cells, such as approved MEK inhibitors trametinib, cobimetinib and selumetinib, and Raf inhibitors sorafenib and dabrafenib. The difficulty lies in complex interactions among signaling pathways during the development and progression of various cancers, and inhibition of a single target may cause compensatory activation of other target pathways, thereby attenuating an anti-cancer effect of a single-target inhibitor. Compared with the single-target inhibitor, a combined use of inhibitors shows a better therapeutic effect in overcoming or delaying drug resistance.

Although progress has been made in the development of inhibitors targeting the Ras/Raf/MEK/ERK signaling pathway, it is apparent that there remains a need to discover and develop a new compound with better efficacy, pharmacokinetic properties and safety, and such researches are of great significance in the field of anti-tumor therapy.

### SUMMARY

The present disclosure discloses an aromatic hydrazide derivative with a structure as shown in general formula (I), and the compound with the structure shows good application prospects in the prevention and/or treatment of a MAPK pathway-related tumor disease.

The present disclosure provides a compound as shown in formula (I), a stereoisomer thereof, a tautomer thereof or a pharmaceutically acceptable salt thereof, 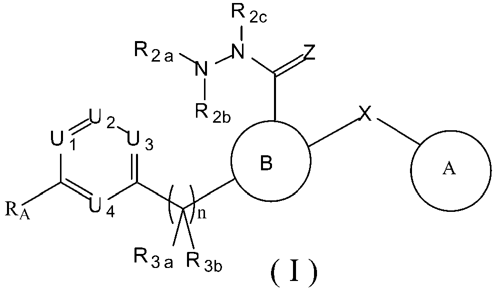
wherein, Z is selected from oxygen or sulfur;
U₁ is selected from nitrogen or CR_{5d}; U₂ is selected from nitrogen or CR₅ₐ; U₃ is selected from nitrogen or CR_{5b}; and U₄ is selected from nitrogen or CR_{5c};
ring B is selected from substituted or unsubstituted aryl or heteroaryl;
R_{A} is selected from -SO₂NRₐR_{b}, -NRₐSO₂NR_{b}R_{c}, -NRₐSO₂R_{b}, -C(O)NRₐR_{b}, -NRₐC(O)NR_{b}R_{c} or -NRₐC(O)R_{b};
each of R₂ₐ, R_{2b} and R_{2c} is independently selected from hydrogen, deuterium, -ORₐ, -SRₐ, C₁₋₆ alkyl, C₂₋₆ alkenyl, C₂₋₆ alkynyl, C₃₋₆ cycloalkyl, 3-10 membered heterocyclyl, C₆₋₁₀ aryl or 5-10 membered heteroaryl, wherein the alkyl, the alkenyl, the alkynyl, the cycloalkyl, the heterocyclyl, the aryl and the heteroaryl are optionally substituted with one or more substituents selected from deuterium, halogen, aldehyde, cyano, amino, hydroxyl, oxo, C₁₋₆ alkyl, C₁₋₆ alkoxy, C₂₋₆ alkenyl, C₂₋₆ alkynyl, C₃₋₆ cycloalkyl, 3-10 membered heterocyclyl, C₆₋₁₀ aryl or 5-10 membered heteroaryl; and
each of R₃ₐ, R_{3b}, R₅ₐ, R_{5b}, R_{5c} and R_{5d} is independently selected from hydrogen, deuterium, halogen, aldehyde, cyano, amino, hydroxyl, C₁₋₆ alkyl, C₁₋₆ deuterated alkyl, C₁₋₆ haloalkyl, C₁₋₆ alkoxy, C₁₋₆ alkylthio, C₂₋₆ alkenyl, C₂₋₆ alkynyl or C₃₋₆ cycloalkyl; or two adjacent substituents together form a substituted or unsubstituted 3-10 membered cyclic structure;
X is selected from -O-, -CRₐR_{b} or -NRₐ;
ring A is selected from substituted or unsubstituted aryl or heteroaryl;
each of Rₐ, R_{b} and R_{c} is independently selected from hydrogen, deuterium, C₁₋₆ alkyl, C₁₋₆ deuterated alkyl, C₁₋₆ alkoxy, C₂₋₆ alkenyl, C₂₋₆ deuterated alkenyl, C₂₋₆ alkynyl, C₂₋₆ deuterated alkynyl, C₃₋₆ cycloalkyl, 3-10 membered heterocyclyl, C₆₋₁₀ aryl or 5-10 membered heteroaryl; and
n is 1, 2, 3 or 4.

The present disclosure provides a compound as shown in formula (II), a stereoisomer thereof, a tautomer thereof or a pharmaceutically acceptable salt thereof, 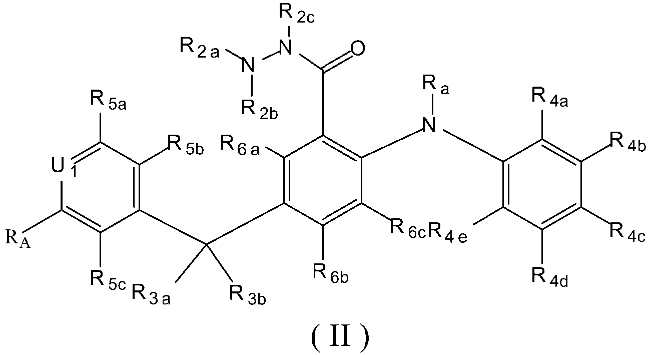
wherein, U₁ is selected from nitrogen or CR_{5d};
R_{A} is selected from -SO₂NRₐR_{b}, -NRₐSO₂NR_{b}R_{c}, -NRₐSO₂R_{b}, -C(O)NRₐR_{b}, -NRₐC(O)NR_{b}R_{c} or -NRₐC(O)R_{b};
each of Rₐ, R_{b} and R_{c} is independently selected from hydrogen, deuterium, C₁₋₆ alkyl, C₁₋₆ deuterated alkyl, C₁₋₆ alkoxy, C₂₋₆ alkenyl, C₂₋₆ deuterated alkenyl, C₂₋₆ alkynyl, C₂₋₆ deuterated alkynyl, C₃₋₆ cycloalkyl, 3-10 membered heterocyclyl, C₆₋₁₀ aryl or 5-10 membered heteroaryl; and
each of R₂ₐ, R_{2b} and R_{2c} is independently selected from hydrogen, deuterium, -ORₐ, -SRₐ, C₁₋₆ alkyl, C₂₋₆ alkenyl, C₂₋₆ alkynyl, C₃₋₆ cycloalkyl, 3-10 membered heterocyclyl, C₆₋₁₀ aryl or 5-10 membered heteroaryl, wherein the alkyl, the alkenyl, the alkynyl, the cycloalkyl, the heterocyclyl, the aryl and the heteroaryl are optionally substituted with one or more substituents selected from deuterium, halogen, aldehyde, cyano, amino, hydroxyl, oxo, C₁₋₆ alkyl, C₁₋₆ alkoxy, C₂₋₆ alkenyl, C₂₋₆ alkynyl, C₃₋₆ cycloalkyl, 3-10 membered heterocyclyl, C₆₋₁₀ aryl or 5-10 membered heteroaryl; and
each of R₃ₐ, R_{3b}, R₄ₐ, R_{4b}, R_{4c}, R_{4d}, R₄ₑ, R₅ₐ, R_{5b}, R_{5c}, R_{5d}, R₆ₐ, R6b and R_{6c} is independently selected from hydrogen, deuterium, halogen, aldehyde, cyano, amino, hydroxyl, C₁₋₆ alkyl, C₁₋₆ deuterated alkyl, C₁₋₆ haloalkyl, C₁₋₆ alkoxy, C₁₋₆ alkylthio, C₂₋₆ alkenyl, C₂₋₆ alkynyl or C₃₋₆ cycloalkyl; or two adjacent substituents together form a substituted or unsubstituted 3-10 membered cyclic structure.

The present disclosure provides a compound as shown in formula (III), a stereoisomer thereof, a tautomer thereof or a pharmaceutically acceptable salt thereof, 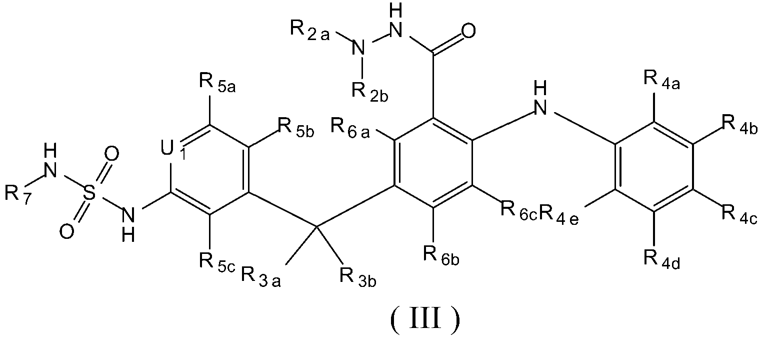
wherein, U₁ is selected from nitrogen or CR_{5d};
each of R₂ₐ and R_{2b} is independently selected from hydrogen, deuterium, -ORₐ, -SRₐ, C₁₋₆ alkyl, C₂₋₆ alkenyl, C₂₋₆ alkynyl, C₃₋₆ cycloalkyl, 3-10 membered heterocyclyl, C₆₋₁₀ aryl or 5-10 membered heteroaryl, wherein the alkyl, the alkenyl, the alkynyl, the cycloalkyl, the heterocyclyl, the aryl and the heteroaryl are optionally substituted with one or more substituents selected from deuterium, halogen, aldehyde, cyano, amino, hydroxyl, oxo, C₁₋₆ alkyl, C₁₋₆ alkoxy, C₂₋₆ alkenyl, C₂₋₆ alkynyl, C₃₋₆ cycloalkyl, 3-10 membered heterocyclyl, C₅₋₁₀ aryl or 5-10 membered heteroaryl;
Rₐ is selected from hydrogen, deuterium, C₁₋₆ alkyl, C₁₋₆ deuterated alkyl, C₂₋₆ alkenyl, C₂₋₆ deuterated alkenyl, C₂₋₆ alkynyl, C₂₋₆ deuterated alkynyl or C₃₋₆ cycloalkyl;
each of R₃ₐ and R_{3b} is independently selected from hydrogen, deuterium, halogen or C₁₋₆ alkyl;
each of R₄ₐ, R4b, R_{4c}, R_{4d}, R₄ₑ, R₅ₐ, R_{5b}, R_{5c}, R₅ₐ, R₆ₐ, R_{6b} and R_{6c} is independently selected from hydrogen, deuterium, halogen, aldehyde, cyano, amino, hydroxyl, C₁₋₆ alkyl, C₁₋₆ deuterated alkyl, C₁₋₆ haloalkyl, C₁₋₆ alkoxy, C₁₋₆ alkylthio, C₂₋₆ alkenyl, C₂₋₆ alkynyl or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl or C₂₋₆ alkynyl.

The present disclosure provides a compound as follows, a stereoisomer thereof, a tautomer thereof or a pharmaceutically acceptable salt thereof,

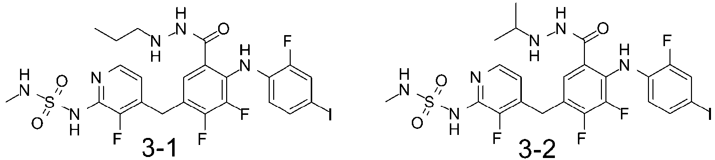

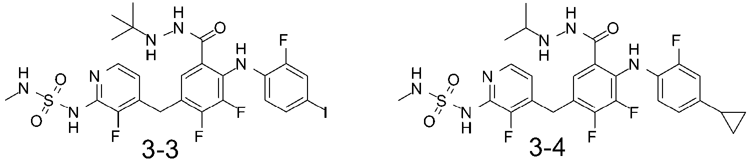

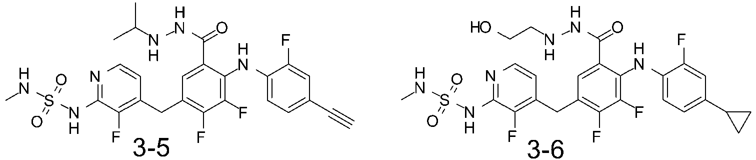

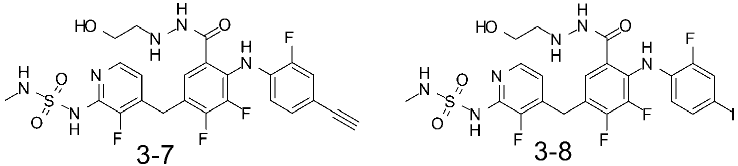

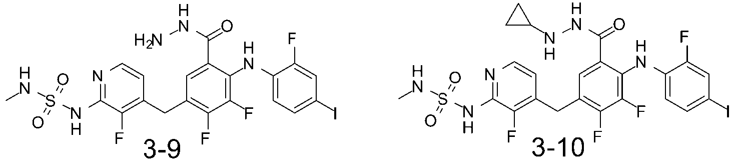

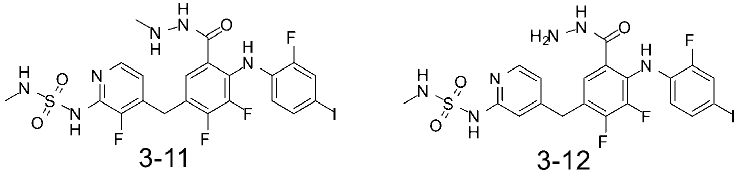

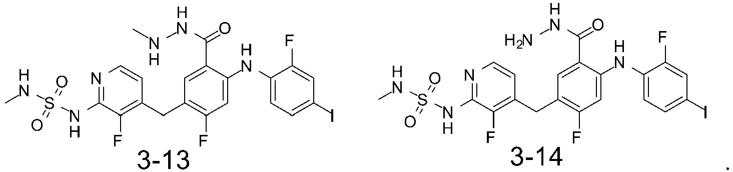

The present disclosure provides a compound as shown in formula (IV-I), a stereoisomer thereof, a tautomer thereof or a pharmaceutically acceptable salt thereof, 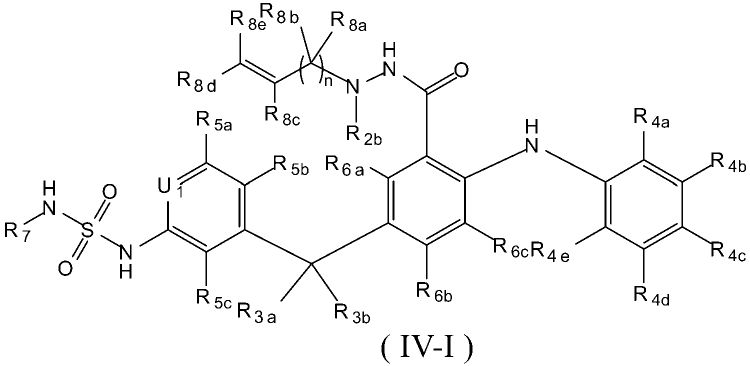
wherein, U₁ is selected from nitrogen or CR_{5d};
R_{2b}, R₃ₐ, R_{3b} are selected from hydrogen, deuterium, halogen, C₁₋₆ alkyl, C₁₋₆ deuterated alkyl or C₁₋₆ haloalkyl;
R₄ₐ, R4b, R_{4c}, R_{4d}, R₄ₑ, R₅ₐ, R_{5b}, R_{5c}, R₅ₐ, R₆ₐ, R_{6b} and R_{6c} are each independently selected from hydrogen, deuterium, halogen, aldehyde, cyano, amino, hydroxyl, C₁₋₆ alkyl, C₁₋₆ deuterated alkyl, C₁₋₆ haloalkyl, C₁₋₆ alkoxy, C₁₋₆ alkylthio, C₂₋₆ alkenyl, C₂₋₆ alkynyl or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl or C₂₋₆ alkynyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl or C₃₋₆ cycloalkyl; and
n is 1, 2, 3 or 4.

The present disclosure provides a compound as shown in formula (IV-II), a stereoisomer thereof, a tautomer thereof or a pharmaceutically acceptable salt thereof, 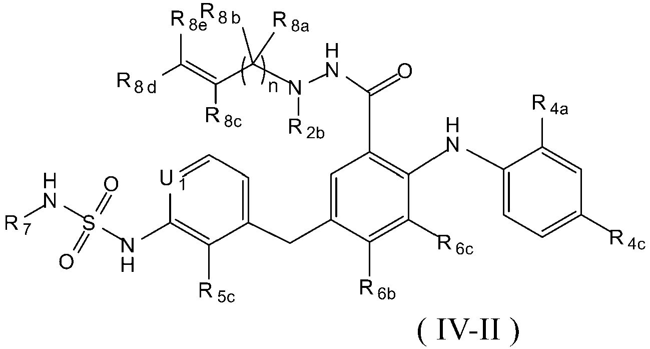
wherein, U₁ is selected from nitrogen or CH;
R_{2b} is selected from hydrogen, deuterium or C₁₋₆ alkyl;
each of R₄ₐ and R_{4c} is independently selected from hydrogen, deuterium, halogen, aldehyde, hydroxyl, C₁₋₆ alkyl, C₁₋₆ alkoxy, C₂₋₆ alkenyl, C₂₋₆ alkynyl or C₃₋₆ cycloalkyl;
each of R_{5c}, R_{6b} and R_{6c} is independently selected from hydrogen, deuterium, halogen or C₁₋₆ alkyl;
R₇ is selected from hydrogen, C₁₋₆ alkyl, C₁₋₆ deuterated alkyl or C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl or C₁₋₆ haloalkyl; and
n is 1, 2, 3 or 4.

The present disclosure provides a compound as shown in formula (IV-III), a stereoisomer thereof, a tautomer thereof or a pharmaceutically acceptable salt thereof, 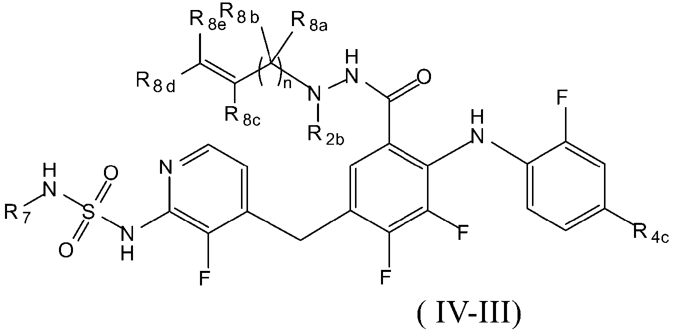
wherein, R_{2b} is selected from hydrogen or C₁₋₆ alkyl;
R_{4c} is selected from halogen, aldehyde, C₁₋₆ alkyl, C₁₋₆ alkoxy, C₂₋₆ alkenyl, C₂₋₆ alkynyl or C₃₋₆ cycloalkyl;
R₇ is selected from C₁₋₆ alkyl, C₁₋₆ deuterated alkyl or C₃₋₆ cycloalkyl;
each of R₈ₐ, R_{8b}, R_{8c}, R_{8d} and R₈ₑ is independently selected from hydrogen, deuterium or C₁₋₆ alkyl; and
n is 1, 2, 3 or 4.

The present disclosure provides a compound as follows, a stereoisomer thereof, a tautomer thereof or a pharmaceutically acceptable salt thereof,

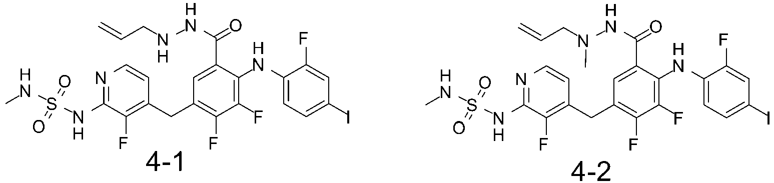

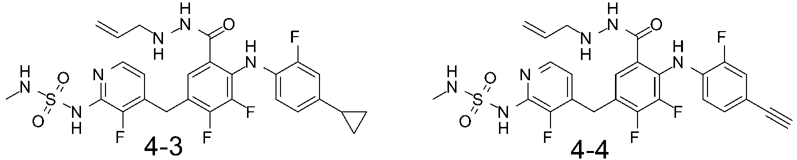

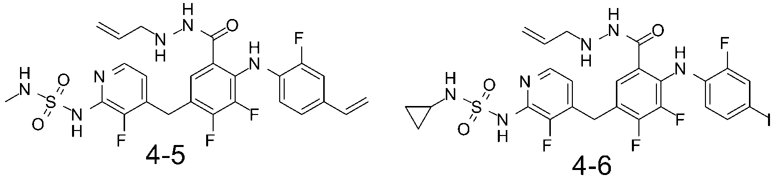

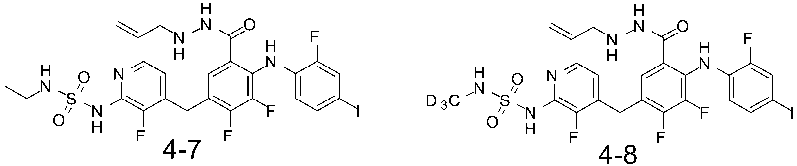

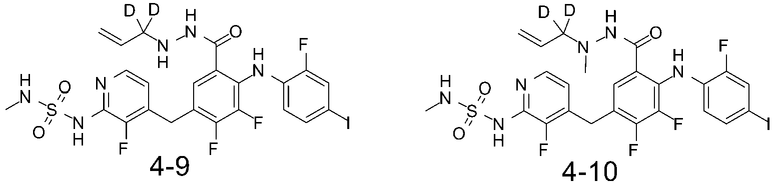

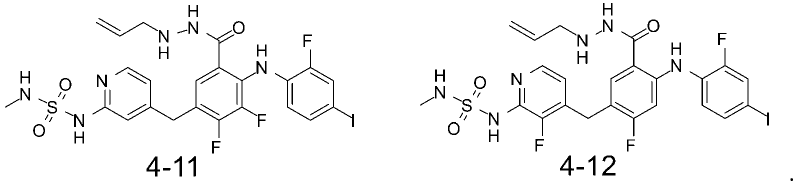

The present disclosure provides a compound as shown in formula (V), a stereoisomer thereof, a tautomer thereof or a pharmaceutically acceptable salt thereof, 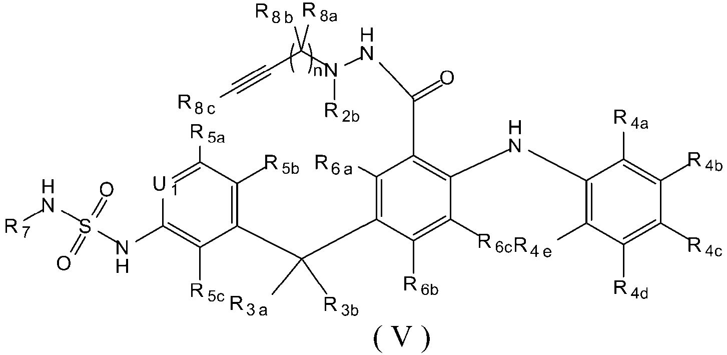
wherein, U₁ is selected from nitrogen or CR_{5d};
R_{2b}, R₃ₐ, R_{3b} are selected from hydrogen, deuterium, halogen, C₁₋₆ alkyl, C₁₋₆ deuterated alkyl or C₁₋₆ haloalkyl;
each of R₄ₐ, R_{4b}, R_{4c}, R_{4d}, R₄ₑ, R₅ₐ, R_{5b}, R_{5c}, R_{5d}, R₆ₐ, R_{6b} and R_{6c} is independently selected from hydrogen, deuterium, halogen, aldehyde, cyano, amino, hydroxyl, C₁₋₆ alkyl, C₁₋₆ deuterated alkyl, C₁₋₆ haloalkyl, C₁₋₆ alkoxy, C₁₋₆ alkylthio, C₂₋₆ alkenyl, C₂₋₆ alkynyl or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl or C₂₋₆ alkynyl;
each of R₈ₐ, R_{8b} and R_{8c} is independently selected from hydrogen, deuterium, halogen, C₁₋₆ alkyl, C₁₋₆ deuterated alkyl, C₁₋₆ haloalkyl or C₃₋₆ cycloalkyl; and
n is 1, 2, 3 or 4.

The present disclosure provides a compound as follows, a stereoisomer thereof, a tautomer thereof or a pharmaceutically acceptable salt thereof,

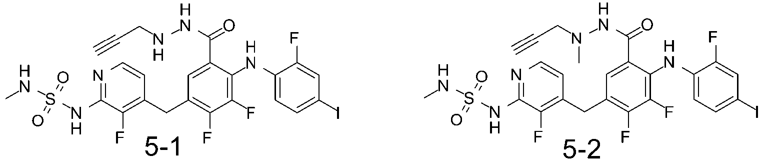

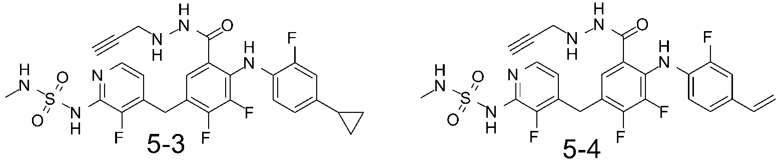

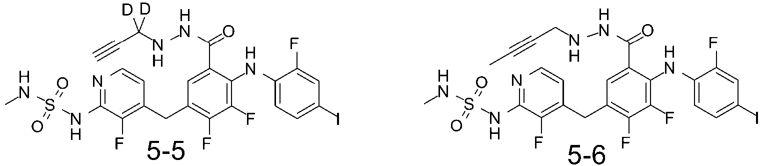

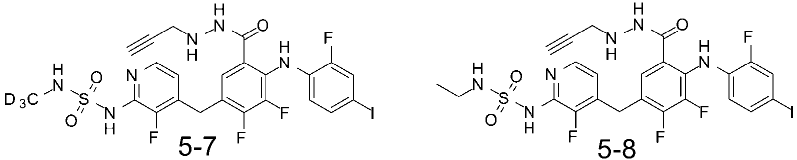

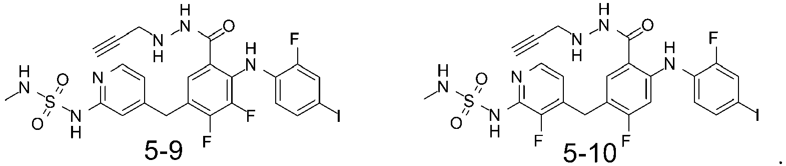

The present disclosure provides a pharmaceutical composition, which comprises a therapeutically effective amount of the compound, the stereoisomer thereof, the tautomer thereof or the pharmaceutically acceptable salt thereof according to any one of the items of the present disclosure, and a pharmaceutically acceptable carrier.

The present disclosure provides use of the compound, the stereoisomer thereof, the tautomer thereof or the pharmaceutically acceptable salt thereof according to any one of the items of the present disclosure, or the pharmaceutical composition of the present disclosure in the manufacture of a medicament for the prevention and/or treatment of a tumor.

The present disclosure provides use of the compound, the stereoisomer thereof, the tautomer thereof or the pharmaceutically acceptable salt thereof according to any one of the items of the present disclosure, or the pharmaceutical composition of the present disclosure in the manufacture of a medicament for the prevention and/or treatment of a MAPK pathway-related disease, wherein the disease particularly refers to a tumor.

According to the use in the present disclosure, the tumor comprises kidney cancer, liver cancer, breast cancer, pancreatic cancer, prostate cancer, melanoma, leukemia, malignant lymphoma, ovarian cancer, head and neck cancer, lung cancer, colorectal cancer, bladder cancer, uterine cancer, and the like.

### Detailed description of the invention

All the technical and scientific terms used in the specification have the same meanings as those commonly understood by those of ordinary skills in the art.

The term "hydrogen" herein refers to H.

The term "deuterium" herein refers to D.

The term "nitrogen" herein refers to N.

The term "oxygen" herein refers to O.

The term "sulfur" herein refers to S.

The term "cyano" herein refers to -CN.

The term "hydroxyl" herein refers to -OH.

The term "halogen" herein refers to -F, -Cl, -Br and -I.

The term "aldehyde" herein refers to -CHO.

The term "amino" or "amine" herein interchangeably refers to a -NR₂ group, wherein each R is, for example, H or a substituent. In some examples, amino is further substituted to form an ammonium ion, such as NR³⁺. An ammonium moiety is specifically included in the definition of "amino" or "amine". The substituent may be, for example, alkyl, deuterated alkyl, alkoxy, cycloalkyl, heterocycloalkyl, aryl, heteroaryl, amide or carboxylate. An R group may be further substituted with one or more (for example, 1 to 4) groups selected from halogen, cyano, alkenyl, alkynyl, alkyl, alkoxy, cycloalkyl, heterocycloalkyl, aryl, heteroaryl, urea, carbonyl, carboxylate, amine and amide.

The term "alkyl" herein refers to a saturated aliphatic hydrocarbon group having 1-15 carbon atoms, preferably 1-10 carbon atoms, and more preferably 1-6 carbon atoms, and the term comprises straight-chain and branched-chain hydrocarbon groups. When the alkyl is preceded by a limitation of carbon atom number, for example, C₁₋₆ alkyl means that the alkyl contains 1-6 carbon atoms. Non-limiting examples of alkyl comprise methyl, ethyl, n-propyl, isopropyl, n-butyl, isobutyl, sec-butyl, tert-butyl, n-pentyl, neopentyl, n-hexyl, and the like. The alkyl herein may be optionally substituted with one or more of the following substituents: deuterium, fluorine, chlorine, bromine, iodine, cyano, nitro, hydroxyl, carboxyl, amino, alkyl, alkoxy, acyl, acyloxy, oxo, amide group, ester group, amine, sulfonyl, sulfinyl, cycloalkyl, heterocyclyl, cycloalkenyl, heterocycloalkyl, alkenyl, alkenoxy, alkynyl, cycloalkoxy, heterocycloalkyloxy, aryloxy, heteroaryloxy, aryl or heteroaryl.

The term "deuterated alkyl" herein refers to an alkyl obtained by substituting the "alkyl" as defined above with deuterium. Non-limiting examples of the deuterated alkyl comprise deuterated methyl, deuterated ethyl, and the like.

The term "haloalkyl" herein refers to an alkyl obtained by substituting the "alkyl" as defined above with halogen, wherein the halogen comprises fluorine, chlorine, bromine, iodine, and the like.

The term "alkoxy" refers to a -ORₐ group, wherein Rₐ is the alkyl as defined above. When the alkoxy is preceded by a limitation of carbon atom number, for example, C₁₋₆ alkoxy means that the alkoxy contains 1-6 carbon atoms. Examples of the alkoxy comprise, but are not limited to, methoxy, ethoxy, isopropoxy, n-propoxy, n-butoxy, tert-butoxy, n-pentoxy, and the like.

Alkenyl is an unsaturated hydrocarbon group containing a carbon-carbon double bond. The term "alkenyl" herein refers to an alkyl group containing a carbon-carbon double bond in a molecule, wherein the alkyl is as defined above. The alkenyl herein has 2 to 10 carbon atoms, preferably 2 to 8 carbon atoms, and more preferably 2 to 6 carbon atoms. When the alkenyl is preceded by a limitation of carbon atom number, for example, C₂₋₆ alkenyl means that the alkenyl contains 2-6 carbon atoms. The alkenyl herein may be optionally substituted with one or more of the following substituents: deuterium, fluorine, chlorine, bromine, iodine, cyano, nitro, hydroxyl, carboxyl, amino, oxo, alkyl, alkoxy, acyl, amide group, ester group, amine, sulfonyl, sulfinyl, cycloalkyl, cycloalkenyl, heterocycloalkyl, cycloalkoxy, mercapto, alkylmercapto, deuterated alkylmercapto, sulfonyl, sulfinyl, silyl, phosphonyl, deuterated alkyl, heterocyclyl, aryl, heteroaryl, alkynyl, alkenyl and arylalkyl. Non-limiting examples of alkenyl comprise, but are not limited to, vinyl, propenyl, allyl, isopropenyl, butenyl, isobutenyl, and the like.

Alkynyl is an unsaturated hydrocarbon group containing a carbon-carbon triple bond. The term "alkynyl" herein refers to an alkyl group containing a carbon-carbon triple bond in a molecule, wherein the alkyl is as defined above. The alkynyl herein has 2 to 10 carbon atoms, preferably 2 to 8 carbon atoms, and more preferably 2 to 6 carbon atoms. When the alkynyl is preceded by a limitation of carbon atom number, for example, C₂₋₆ alkynyl means that the alkynyl contains 2-6 carbon atoms. The alkynyl may be optionally substituted with one or more of the following substituents: deuterium, fluorine, chlorine, bromine, iodine, cyano, nitro, hydroxyl, carboxyl, amino, alkyl, oxo, alkoxy, acyl, amide group, ester group, amine, sulfonyl, sulfinyl, cycloalkyl, cycloalkenyl, heterocycloalkyl, cycloalkoxy, mercapto, alkylmercapto, deuterated alkylmercapto, sulfonyl, sulfinyl, silyl, phosphonyl, deuterated alkyl, heterocyclyl, aryl, heteroaryl, alkynyl, alkenyl and arylalkyl. Non-restrictive examples of the alkynyl comprise, but are not limited to, ethynyl, 1-propynyl, 2-propynyl, 1-, 2- or 3-butynyl, and the like.

The term "cycloalkyl" refers to a stable non-aromatic monocyclic or polycyclic hydrocarbon group consisting of carbon atoms and hydrogen atoms only, which may comprise a fused ring system, a spiro ring system or a bridged ring system having 3 to 15 carbon atoms, preferably 3 to 10 carbon atoms, more preferably 3 to 8 carbon atoms, and most preferably 3 to 6 carbon atoms, and is saturated or unsaturated and may be linked to the rest of the molecule by a single bond via any suitable carbon atom. When the cycloalkyl is preceded by a limitation of carbon atom number, for example, C₃₋₆ cycloalkyl means that the cycloalkyl contains 3-6 carbon atoms. Non-limiting examples of the cycloalkyl comprise, but are not limited to, cyclopropyl, cyclobutyl, cyclopentyl, cyclohexyl, cycloheptyl, cyclooctyl, 1H-indenyl, 2,3-dihydroindenyl, 1,2,3,4-tetrahydro-naphthyl, 5,6,7,8-tetrahydro-naphthyl, 8,9-hydro-7H-benzocyclohepten-6-yl, 6,7,8,9-tetrahydro-5H-benzocycloheptenyl, 5,6,7,8,9,10-hexahydro-benzocyclooctenyl, fluorenyl, bicyclo[2.2.1]heptyl, 7,7-dimethyl-bicyclo[2.2.1]heptyl, bicyclo[2.2.1]heptenyl, bicyclo[2.2.2]octyl, bicyclo[3.1.1]heptyl, bicyclo[3.2.1]octyl, bicyclo[2.2.2]octenyl, bicyclo[3.2.1]octenyl, adamantyl, octahydro-4,7-methylene-1H-indenyl, octahydro-2,5-methylene-cyclopentadienyl, and the like. The cycloalkyl herein may be optionally substituted with one or more of the following substituents: deuterium, fluorine, chlorine, bromine, iodine, cyano, nitro, hydroxyl, oxo, carboxyl, amino, alkyl, alkoxy, acyl, amide group, ester group, amine, sulfonyl, sulfinyl, cycloalkyl, heterocyclyl, cycloalkenyl, heterocycloalkyl, alkenyl, alkynyl, cycloalkoxy, aryl and heteroaryl.

The term "heterocyclyl" refers to a substituted or unsubstituted and saturated or unsaturated aromatic ring or non-aromatic ring containing at least 1 to 5 heteroatoms selected from N, O or S. The aromatic ring or non-aromatic ring may be a 3-10 membered monocyclic ring, or a 4-20 membered spirocyclic ring, fused ring or bridged ring, preferably a 3-12 membered heterocyclic ring. N and S optionally substituted in the ring of the heterocyclyl may be oxidized into various oxidation states. Non-limiting examples of heterocyclyl comprise oxiranyl, oxetanyl, oxolanyl, oxanyl, oxepanyl, oxocanyl, aziridinyl, azetidinyl, azolidinyl, azinanyl, azirinyl, 1,3-dioxolanyl, 1,4-dioxolanyl, 1,3-dithiolanyl, 1,3-dioxanyl, 1,3-dithianyl, azepinyl, morpholinyl, piperazinyl, pyridinyl, furanyl, thiophenyl, pyrrolyl, pyranyl, N-alkylpyrrolyl, pyrimidinyl, pyrazinyl, pyridazinyl, imidazolyl, piperidinyl, thiomorpholinyl, dihydropyranyl, thiadiazolyl, oxazolyl, oxadiazolyl, pyrazolyl, 1,4-dioxadienyl, and the like. The heterocyclyl herein may be optionally substituted with one or more of the following substituents: deuterium, fluorine, chlorine, bromine, iodine, cyano, nitro, hydroxyl, carboxyl, amino, alkyl, alkoxy, acyl, amide group, ester group, amine, sulfonyl, sulfinyl, cycloalkyl, heterocyclyl, cycloalkenyl, heterocycloalkyl, alkenyl, alkynyl, cycloalkoxy, aryl and heteroaryl.

The term "aryl" herein refers to a 6-14 membered full-carbon monocyclic or fused polycyclic (for example, a ring sharing a pair of adjacent carbon atoms) group with a conjugated π-electron system, preferably a 6-10 membered ring, for example, phenyl and naphthyl, more preferably phenyl. A ring of the aryl may be fused on a ring of heteroaryl, heterocyclyl or cycloalkyl, wherein a ring linked to the parent structure is the ring of the aryl. The aryl herein may be substituted or unsubstituted, and in the case of being substituted, a substituent is preferably one or more of the following groups: deuterium, fluorine, chlorine, bromine, iodine, cyano, nitro, hydroxyl, carboxyl, amino, alkyl, alkoxy, acyl, amide group, ester group, sulfonyl, sulfinyl, cycloalkyl, heterocycloalkyl, cycloalkenyl, alkenyl, alkynyl, heterocyclyl, cycloalkoxy, aryl and heteroaryl.

The term "heteroaryl" herein refers to an aromatic group consisting of 5 to 10 atoms and containing at least one heteroatom selected from N, O or S. This term may have a single ring (non-limiting examples comprise furan, thiophene, imidazole, triazole, pyrazole, pyridine, pyrazine, oxazole, thiazole, and the like) or multiple fused rings (non-limiting examples comprise benzothiophene, benzofuran, indole, isoindole, and the like), wherein the fused ring may or may not be an aromatic group containing heteroatoms, assuming that a linking point is an atom of an aromatic heteroaryl group. The heteroaryl herein may be optionally substituted with one or more of the following substituents: deuterium, fluorine, chlorine, bromine, iodine, cyano, nitro, hydroxyl, amino, oxo, alkyl, alkoxy, acyl, acyloxy, amide group, ester group, amine, sulfonyl, sulfinyl, cycloalkyl, heterocycloalkyl, cycloalkenyl, alkenyl, alkynyl, heterocyclyl, cycloalkoxy, aryl and heteroaryl.

The present disclosure further comprises isotopically labeled compounds of the present disclosure, such as having the same structure as disclosed above, but one or more atoms in this structure are substituted with atoms with the same proton number but different neutron number. Examples of isotopes combined in the compounds of the present disclosure comprise isotopes of hydrogen, carbon, nitrogen, oxygen, sulfur, fluorine, chlorine and iodine, such as ²H, ³H, ¹³C, ¹⁴C, ¹⁵N, ¹⁸O, ¹⁷O, ³⁵S, ¹⁸F , ³⁶Cl and ¹³¹I respectively. The compounds of the present disclosure, their stereoisomers, tautomers or pharmaceutically acceptable salts, and the compounds in the above forms containing the above isotopes and/or other atomic isotopes are all included within the scope of the present disclosure. Some isotopically labeled compounds of the present disclosure, such as those compounds labeled by ³H or ¹⁴C, may be used in a drug tissue distribution experiment. Therefore, these ³H or ¹⁴C isotopes are particularly preferred because of easy preparation and detection. Some compounds of the present disclosure substituted with heavier isotopes, such as ²H and ¹⁸O, have some therapeutic advantages due to better metabolic stability, such as an increased in-vivo half-life period, a reduced dosage, and other comprehensive performances. Therefore, ²H and ¹⁸O are also preferred in some cases.

The term "optional" or "optionally" means that the subsequently described event or situation may, but does not necessarily, occur, and the description comprises the case in which the event or situation occurs and the case in which the event or situation does not occur.

The term "compound of the present disclosure" (unless otherwise specified) refers to the compounds as shown in formula (I)-formula (V) and all their pure and mixed stereoisomers, geometric isomers, tautomers, solvates, hydrates, prodrugs, isotopically labeled compounds and any pharmaceutically acceptable salts. The solvate of the compound of the present disclosure refers to a compound combined with stoichiometric and non-stoichiometric solvents or a salt thereof, such as a hydrate, an ethanolate and a methanolate. The compound may also exist in one or more crystalline states, such as serving as a cocrystal and a polymorph, or may exist in an amorphous solid state. All such forms are covered by the claims.

The term "pharmaceutically acceptable" means that a substance or composition must be chemically and/or toxicologically compatible with other ingredients constituting the preparation and/or mammals treated with the preparation.

The term "optionally substituted with ..." means that the structure is unsubstituted or substituted with one or more substituents according to the present disclosure. The term "substituted" herein means that any group is monosubstituted or polysubstituted with a specified substituent to an extent that such monosubstitution or polysubstitution (including multi-substitution in the same part) is chemically permitted, and each substituent may be located in any available position on the group and may be linked by any available atom on the substituent. "Any available position" refers to any position on the group that may be chemically obtained by a method known in the art or a method taught herein, and does not produce excessively unstable molecules. When there are two or more substituents on any group, each substituent is independently defined from any other substituent, so that the substituents may be the same or different.

In various parts of the specification, the substituents of the compounds of the present disclosure are disclosed in the form of groups or ranges, which specifically means that the present disclosure includes a sub-combination of each member or each individual in the member of such groups and ranges. For example, the term "C₁₋₆ alkyl" specifically means that methyl, ethyl, C₃ alkyl, C₄ alkyl, C₅ alkyl and C₆ alkyl are separately disclosed.

The term "stereoisomer" herein refers to a compound with different chirality having one or more stereocenters. The stereoisomer comprises an enantiomer, a diastereomer, a conformational isomer (rotational isomer), a geometric isomer (cis/trans) isomer, an atropisomer, and the like.

The term "tautomer" herein refers to a structural isomer with different energy that may achieve mutual conversion by crossing a low energy barrier. For example, a proton tautomer includes a tautomer of interconversion through protolysis, such as an enol-ketone tautomer and an imine-enamine tautomer, or a tautomeric form containing a heteroaryl group linked to a ring atom of ring-NH-moiety and ring=N-moiety, such as pyrazole, imidazole, benzimidazole, triazole and tetrazole. A valence tautomer comprises a tautomer obtained grom interconversion through recombination of some bonding electrons.

The compounds of the present disclosure may be used in the form of salts, such as the "pharmaceutically acceptable salts" derived from inorganic or organic acids. These salts include, but are not limited to: acetate, adipate, alginate, citrate, aspartate, benzoate, benzenesulfonate, bisulfate, butyrate, camphorate, camphorsulfonate, digluconate, cyclopentanepropionate, sodium dodecylbenzenesulfonate sulfate, ethane sulfonate, glucosyl heptanoate, glycerophosphate, hemisulfate, heptanoate, caproate, fumarate, hydrochloride, hydrobromide, hydroiodide, 2-hydroxyethylsulfonate, lactate, maleate, methanesulfonate, hydrochloride, 2-naphthalenesulfonate, oxalate, pectate, sulfate, 3-phenylpropionate, picrate, pivalate, propionate, succinate, tartrate, thiocyanate, p-toluenesulfonate, quinate, and the like.

In the present application, "tumor" comprises, but is not limited to: hematological and lymphoid cancers such as leukemia, malignant lymphoma, multiple myeloma and myelodysplastic syndrome; and solid tumors such as brain tumor, neural tumor, head and neck cancer, esophageal cancer, gastric cancer, colorectal cancer, lung cancer, thyroid cancer, breast cancer, renal cancer, gallbladder cancer, pancreatic cancer, liver cancer, prostate cancer, uterine cancer, testicular cancer, ovarian cancer, renal cell carcinoma, bladder cancer, renal pelvic and ureteral cancer, melanoma and skin cancer.

### DESCRIPTION OF EXEMPLARY EMBODIMENTS

The present disclosure is further described hereinafter with reference to embodiments, but the present disclosure is not limited by the embodiments. Throughout the present application, multiple embodiments of the compounds and methods of the present disclosure are mentioned herein. The present disclosure is not limited to these embodiments, and the following embodiments only provide the methods for practicing the present disclosure and do not limit the scope of the present disclosure in any way.

The compounds provided by the present disclosure may be prepared by standard synthesis methods well known in the art, and the specification provides general methods for preparing the compounds of the present disclosure. Starting materials are usually commercially available, such as those purchased through Alfa Aesar^{®}, Sigma-Aldrich^{®}, TCI and other companies, or those prepared by methods well known to those skilled in the art.

A compound as shown in formula (III), a stereoisomer thereof, a tautomer thereof or a pharmaceutically acceptable salt thereof in the present disclosure may be prepared by the following process.

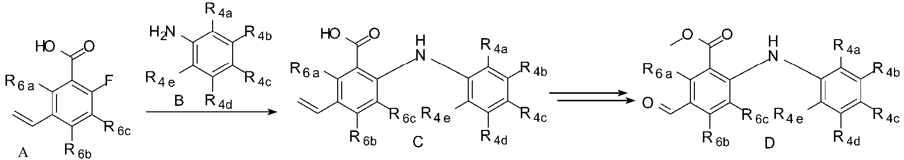

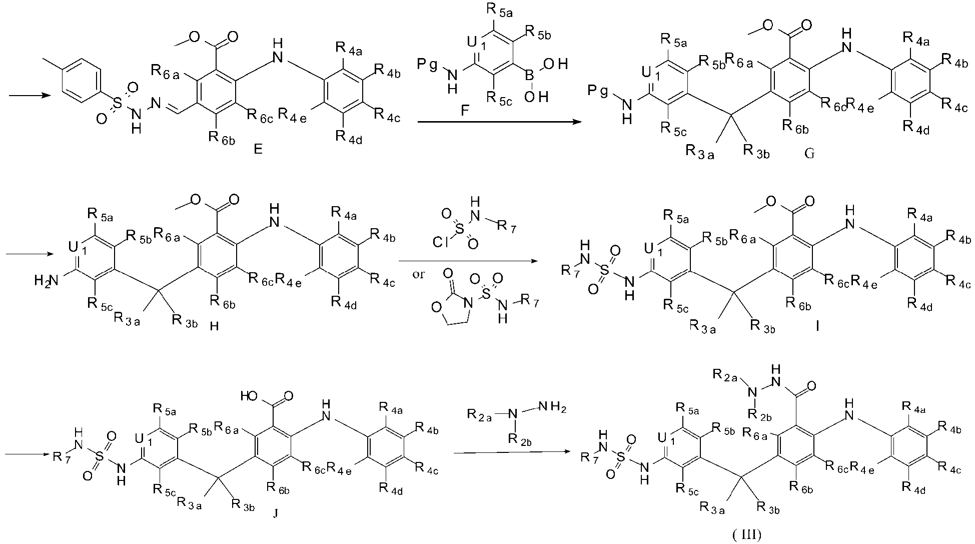

The compound as shown in formula (III) of the present disclosure may be prepared according to the following process: under alkaline conditions, a fluorobenzene derivative A is allowed to react with an aniline derivative B to form a diphenylamine derivative C; diphenylamine derivative C is subjected to an esterification reaction and an oxidation reaction to form an aldehyde derivative D; aldehyde derivative D is allowed to react with an arylsulfonylhydrazide to form a hydrazone derivative E; under alkaline conditions, hydrazone derivative E is allowed to react with an arylboronic acid derivative F to form a compound G; under acidic conditions, compound G is deprotected to form an ester derivative H; under alkaline conditions, the ester derivative H is allowed to react with a corresponding sulfonyl compound to prepare a compound I; compound I is hydrolyzed in the presence of a hydroxide to form a benzoic acid derivative J; and in the presence of a condensing agent, benzoic acid derivative J is allowed to react with corresponding hydrazine or hydrazine hydrochloride to prepare the compound as shown in general formula III. The definition of each substituent is as described above.

The compounds of the present disclosure and the corresponding preparation methods are further explained and enumerated hereinafter through the embodiments and the preparations. It should be understood that, although typical or preferred reaction conditions (such as reaction temperature and time, molar ratios of reactants, and reaction solvents) are given in specific embodiments, other reaction conditions may also be used by those skilled in the art. The optimum reaction conditions may vary depending on a specific reaction substrate or solvent used, but the conditions may be determined by those skilled in the art through routine optimization.

Structures of the compounds in the following embodiments are characterized by nuclear magnetic resonance (NMR) and/or mass spectrometry (MS). The compounds are dissolved in appropriate deuterated reagents, using a Bruker Ascend 400 MHz NMR spectrometer, and analyzed by ¹H-NMR with TMS as an internal standard at ambient temperature. An NMR chemical shift (δ) is in a unit of ppm, and the following abbreviations are used: s, single peak; d, double peak; t, triple peak; q, quadruple peak; m, multiple peaks; and brs, broad single peak.

The starting materials in reaction, the intermediates and the compounds of the embodiments may be separated and purified by precipitation, filtration, crystallization, evaporation, distillation, chromatography (such as silica gel column chromatography and preparative chromatography), and other technologies.

### Preparation of intermediates

### Preparation of intermediate 1

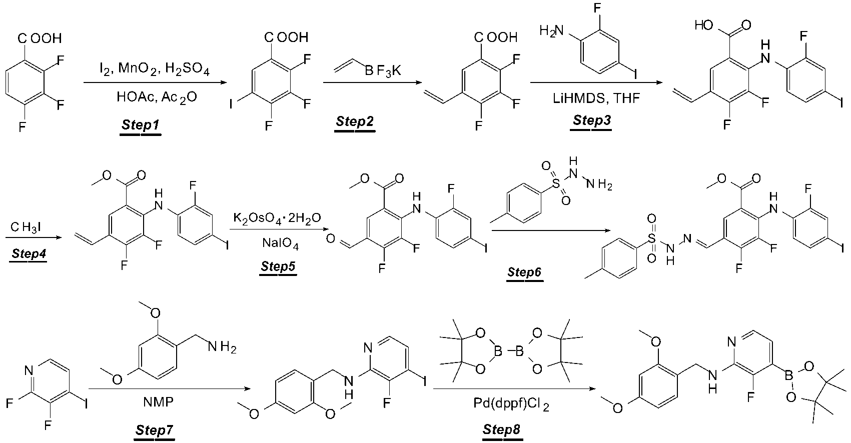

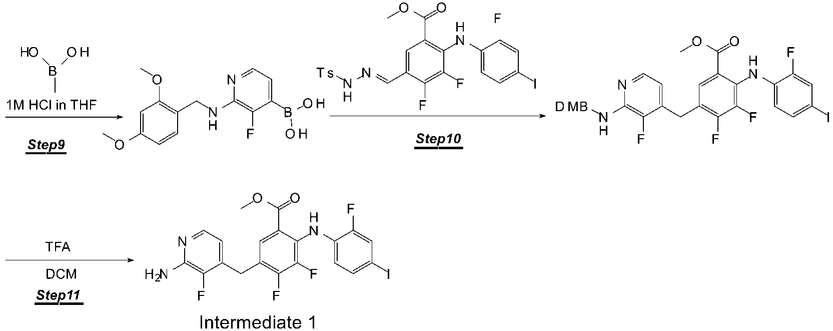

### Step 1: preparation of 2,3,4-trifluoro-5-iodobenzoic acid

Acetic acid (928 mL) and acetic anhydride (538.0 g) were added into a 3 L three-necked flask, and cooled to 0°C in an ice bath. Concentrated sulfuric acid (2116.0 g) was slowly dropwise added to the system while maintaining an internal temperature of the system not higher than 40°C. After finishing the dropwise addition, the system was cooled to room temperature again. Subsequently, 2,3,4-trifluorobenzoic acid (115.0 g), iodine (66.9 g) and manganese dioxide (68.7 g) were added. After finishing the addition, the system was heated to 50°C and allowed to react for 3 hours. After stopping heating and cooling to room temperature, iodine (66.9 g) and manganese dioxide (68.7 g) were added additionally, and subsequently, the system was heated to 50 °C to react for 3 hours. After stopping heating and cooling the system to room temperature, iodine (33.4 g) and manganese dioxide (34.4 g) were additionally added, and subsequently, the system was heated to 50°C again and allowed to react for 21 hours. After stopping the reaction and cooling to room temperature, the system was slowly poured into ice water for quenching. The mixture was extracted with dichloromethane, and the organic phases were combined, washed with an aqueous sodium sulfite solution and saturated brine respectively, and dried with anhydrous sodium sulfate. After concentrating the organic phases, n-heptane was added, and the mixture was stirred in an ice bath to precipitate a large amount of solid. The mixture was subjected to suction filtration, and the filter cake was washed with n-heptane to obtain 178.1 g of white solid as a product. ESI-MS: [M - H]⁻ = 300.9.

### Step 2: preparation of 2,3,4-trifluoro-5-vinylbenzoic acid

2,3,4-trifluoro-5-iodobenzoic acid (89.0 g), potassium vinyltrifluoroborate (47.4 g), potassium phosphate (93.8 g), methanol (1780 mL), water (890 mL) and [1,1'-bis(diphenylphosphino)ferrocene]dichloropalladium(II) (21.6 g) were sequentially added into a 3 L three-necked flask. The system was subjected to nitrogen purging, and heated to 60°C and allowed to react for 3 hours. After cooling to room temperature, the system was filtered through diatomaceous earth, and the filtrate was collected and concentrated. 1 M HCl was added to adjust the pH value of the system to be 4-5. Subsequently, the mixture was extracted with methyl tert-butyl ether, and the organic phases were combined, washed with saturated brine, dried and concentrated. N-heptane and dichloromethane were added to the concentrate, stirred at room temperature for 2 hours and filtered, and the filter cake was washed with n-heptane to obtain 48.2 g of white solid. ESI-MS: [M - H]⁻ = 201.0.

### Step 3: preparation of 3,4-difluoro-2-((2-fluoro-4-iodophenyl)amino)-5-vinylbenzoic acid

2,3,4-trifluoro-5-vinylbenzoic acid (65.0 g), 2-fluoro-4-iodoaniline (76.3 g) and tetrahydrofuran (1300 mL) were added into a 3 L three-necked flask. Subsequently, the system was subjected to nitrogen purging, and cooled to -15°C to -20°C. Lithium bis(trimethylsilyl)amide (1 M, 965 mL) was slowly dropwise added to the system while ensuring that the temperature of the system was not higher than -15°C. After finishing the dropwise addition, the system was slowly allowed to warm to room temperature, and subsequently, 1 M hydrochloric acid was added to adjust the pH value of the system to be 1-2. The mixture was extracted with methyl tert-butyl ether, and the organic phases were combined, washed with saturated brine, dried and concentrated. N-heptane was slowly added to the concentrate to precipitate a solid, and the mixture was filtered to obtain 99.0 g of off-white solid. ESI-MS: [M - H]⁻ = 418.0.

### Step 4: preparation of methyl 3,4-difluoro-2-((2-fluoro-4-iodophenyl)amino)-5-vinylbenzoate

3,4-difluoro-2-((2-fluoro-4-iodophenyl)amino)-5-vinylbenzoic acid (20.0 g) and N,N-dimethylformamide (200 mL) were added into a 1 L three-necked flask, and the system was stirred at room temperature until a clear solution was obtained. Potassium carbonate (7.9 g) and methyl iodide (8.1 g) were added to the system, and subsequently, the system was allowed to react at room temperature for 12 hours. Suspended matters in the system were removed by filtration, and the filtrate was collected. The filtrate was slowly dropwise added to water to precipitate a solid. After finishing the dropwise addition, the system was continuously stirred for 1 hour. The mixture was subjected to suction filtration, and the filter cake was washed with 250 mL of ethanol to obtain 15.1 g of light yellow solid.

### Step 5: preparation of methyl 3,4-difluoro-2-((2-fluoro-4-iodophenyl)amino)-5-formylbenzoate

Methyl 3,4-difluoro-2-((2-fluoro-4-iodophenyl)amino)-5-vinylbenzoate (11.6 g) and tetrahydrofuran (200 mL) were added into a 1 L three-necked flask, and stirred in an ice bath until a clear solution was obtained. Potassium osmate (VI) dihydrate (494 mg) was added to the system, and continuously stirred in an ice bath for reaction. After 15 minutes, water (200 mL), sodium periodate (17.2 g) and 2,6-dimethylpyridine (2.9 g) were added to the system, and the system was allowed to warm to room temperature and stirred to react for 4 hours. The reaction system was poured into a saturated aqueous sodium sulfite solution and extracted with ethyl acetate, and the organic phases were combined, washed with saturated brine, and concentrated to obtain a solid crude product. The crude product was washed with anhydrous ethanol, and filtered to obtain 9.0 g of light brown solid. ¹H NMR (400 MHz, DMSO-*d*₆): δ 10.00 (s, 1H), 9.52 (s, 1H), 8.21 (dd, *J*₁ = 7.2 Hz, *J*₂ = 1.2 Hz, 1H), 7.71 (dd, *J*₁ = 10.4 Hz, *J*₂ = 1.6 Hz, 1H), 7.54-7.51 (m, 1H), 7.13-7.07 (m, 1H), 3.86 (s, 3H). ESI-MS: [M + H]⁺ = 436.0.

### Step 6: preparation of methyl (E)-3,4-difluoro-2-((2-fluoro-4-iodophenyl)amino)-5-((2-(p-tolylsulfonyl)hydrazono)meth yl)benzoate

Methyl 3,4-difluoro-2-((2-fluoro-4-iodophenyl)amino)-5-formylbenzoate (50.7 g), p-toluenesulfonyl hydrazide (21.7 g) and ethanol (1500 mL) were added into a 2 L three-necked flask, and the system was heated to 50°C and allowed to react for 5 hours. Subsequently, the system was cooled to 0°C in an ice bath to precipitate a large amount of solid. The mixture was subjected to suction filtration, and the filter cake was washed with ethanol to obtain 53.1 g of light yellow solid. ¹H NMR (400 MHz, DMSO-*d*₆): δ 11.76 (s, 1H), 8.97 (s, 1H), 8.00-7.99 (m, 2H), 7.75 (d, *J =* 8.0 Hz, 2H), 7.64 (dd, *J*₁ = 10.4 Hz, *J*₂ *=* 1.6 Hz, 1H), 7.43 (d, *J* = 8.4 Hz, 3H), 6.92-6.89 (m, 1H), 3.85 (s, 3H), 2.37 (s, 3H). ESI-MS: [M + H]⁺ = 604.0.

### Step 7: preparation of N-(2,4-dimethoxybenzyl)-3-fluoro-4-iodopyridin-2-amine

2,3-difluoro-4-iodopyridine (45.0 g), (2,4-dimethoxyphenyl)methylamine (78.0 g), N-methylpyrrolidone (675 mL) and triethylamine (56.7 g) were added into a 1 L single-necked flask, and the system was subjected to nitrogen purging. The system was heated to 100°C and allowed to react for 2 hours. After cooling to room temperature, the system was poured into water. The mixture was extracted with methyl tert-butyl ether, and the organic phases were combined, washed with saturated brine, dried and concentrated to obtain a crude product. The concentrate was slurried with methyl tert-butyl ether / petroleum ether (1/4) to obtain 56.8 g of off-white solid. ¹H NMR (400 MHz, DMSO-*d*₆): δ 7.45 (d, *J =* 5.6 Hz, 1H), 7.11-7.08 (m, 1H), 7.03 (d, *J =* 8.4 Hz, 1H), 6.89-6.86 (m, 1H), 6.54 (d, *J =* 2.4 Hz, 1H), 6.43 (dd, *J*₁ = 8.4 Hz, *J*₂ = 2.4 Hz, 1H), 4.44 (d, *J =* 6.0 Hz, 2H), 3.80 (s, 3H), 3.72 (s, 3H).

### Step 8: preparation of N-(2,4-dimethoxybenzyl)-3-fluoro-4-(4,4,5,5-tetramethyl-1,3,2-dioxaborolan-2-yl)pyridin-2-amine

N-(2,4-dimethoxybenzyl)-3-fluoro-4-iodopyridin-2-amine (56.5 g), bis(pinacolato)diboron (147.8 g), potassium pivalate (44.9 g), toluene (1400 mL), dimethyl sulfoxide (20 mL) and [1,1'-bis(diphenylphosphino)ferrocene]dichloropalladium(II) (10.6 g) were added into a 2 L three-necked flask, and the system was subjected to nitrogen purging. Subsequently, the system was heated to 55°C and allowed to react for 15 hours. After cooling to room temperature, the system was poured into water. The mixture was extracted with ethyl acetate, and the organic phases were combined, dried with anhydrous sodium sulfate and concentrated. Subsequently, the concentrate was diluted with n-hexane, dried and filtered through diatomaceous earth, and the filtrate was concentrated to obtain a product. The product in this step was directly used in the next step of reaction without further purification. ¹H NMR (400 MHz, DMSO-*d*₆): δ 7.75 (dd, *J*₁ = 4.8 Hz, *J*₂ = 0.8 Hz, 1H), 7.01 (d, *J =* 8.4 Hz, 1H), 6.87-6.84 (m, 1H), 6.59-6.56 (m, 1H), 6.54 (d, *J =* 2.4 Hz, 1H), 6.42 (dd, *J*₁ = 8.4 Hz, *J*₂ = 2.4 Hz, 1H), 4.45 (d, *J =* 6.0 Hz, 2H), 3.80 (s, 3H), 3.72 (s, 3H), 1.30 (s, 12H).

### Step 9: preparation of (2-((2,4-dimethoxybenzyl)amino)-3-fluoropyridin-4-yl)boronic acid

N-(2,4-dimethoxybenzyl)-3-fluoro-4-(4,4,5,5-tetramethyl-1,3,2-dioxaborolan-2-yl)pyr idin-2-amine and THF (500 mL) were added into a 2 L single-necked flask, and stirred until a clear solution was obtained. 1 M aqueous hydrochloric acid solution was slowly added to the system, and stirred at room temperature and allowed to react for 1 hour. The mixture was extracted with petroleum ether, and an aqueous phase was collected. Methylboric acid (17.4 g) was added to the aqueous phase, and stirred at room temperature and allowed to react for 1 hour. Subsequently, the mixture was extracted with a mixed solution of ethyl acetate/petroleum ether (3/1), and an aqueous phase was collected. Ethyl acetate was added to the aqueous phase, an aqueous sodium bicarbonate solution was added to adjust the pH value of the system to be 8, and an organic phase was collected, washed with water, dried and concentrated to obtain 37.0 g of off-white solid. ESI-MS: [M + H]⁺ =307.1.

### Step 10: preparation of methyl 5-((2-((2,4-dimethoxybenzyl)amino)-3-fluoropyridin-4-yl)methyl)-3,4-difluoro-2-((2-fluor o-4-iodophenyl)amino)benzoate

(2-((2,4-dimethoxybenzyl)amino)-3-fluoropyridin-4-yl)boronic acid (15.0 g), methyl (E)-3,4-difluoro-2-((2-fluoro-4-iodophenyl)amino)-5-((2-(p-tolylsulfonyl)hydrazono)meth yl)benzoate (35.4 g), potassium carbonate (8.1 g) and toluene (600 mL) were added into a 1 L single-necked flask, and the system was subjected to nitrogen purging. The system was heated to 100°C and allowed to react for 5 hours. After cooling to room temperature, a saturated aqueous ammonium chloride solution was added to the system. The mixture was extracted with ethyl acetate, and the organic phases were combined and dried with anhydrous sodium sulfate. The mixture was filtered and subjected to rotary evaporation to remove the solvent. Subsequently, the crude product was purified by silica gel column chromatography to obtain 17.9 g of light yellow liquid. ¹H NMR (400 MHz, DMSO-*d*₆): δ 8.65 (s, 1H), 7.73 (d, *J =* 7.2 Hz, 1H), 7.68 (d, *J =* 5.2 Hz, 1H), 7.62 (dd, *J*₁ = 10.8 Hz, *J*₂ *=* 3.0 Hz, 1H), 7.39 (dd, *J*₁ = 8.4 Hz, *J*₂ = 0.8 Hz, 1H), 7.03 (d, *J=* 8.4 Hz, 1H), 6.92-6.88 (m, 1H), 6.80-6.75 (m, 1H), 6.54 (d, *J* = 2.4 Hz, 1H), 6.42 (dd, *J*₁ = 8.4 Hz, *J*₂ = 2.4 Hz, 1H), 6.38 (t, *J* = 4.8 Hz, 1H), 4.44 (d, *J* = 6.0 Hz, 2H), 4.01 (s, 2H), 3.80 (d, *J* = 1.2 Hz, 6H), 3.72 (s, 3H). ESI-MS: [M + H]⁺ = 682.1.

### Step 11: preparation of methyl 5-((2-amino-3-fluoropyridin-4-yl)methyl)-3,4-difluoro-2-((2-fluoro-4-iodophenyl)amino)b enzoate

Methyl 5-((2-((2,4-dimethoxybenzyl)amino)-3-fluoropyridin-4-yl)methyl)-3,4-difluoro-2-((2-fluor o-4-iodophenyl)amino)benzoate (10.0 g) and dichloromethane (150 mL) were added into a 500 mL single-necked flask, and stirred at room temperature until a clear solution was obtained. Subsequently, the system was placed in an ice bath, and trifluoroacetic acid (150 mL) was slowly added to the system. After finishing the addition, the system was allowed to warm to room temperature and stirred to react for 1.5 hours. A saturated aqueous sodium carbonate solution was slowly added to the system to adjust the pH value of the system to be 8. The mixture was extracted with dichloromethane, and the organic phases were combined and dried with anhydrous sodium sulfate. The mixture was filtered and subjected to rotary evaporation to remove the solvent. Subsequently, the crude product was purified by silica gel column chromatography to obtain 5.8 g of light yellow solid. ¹H NMR (400 MHz, DMSO-*d*₆): δ 8.64 (s, 1H), 7.71 (d, *J =* 7.2 Hz, 1H), 7.67 (d, *J =* 5.2 Hz, 1H), 7.62 (dd, *J*₁ = 10.8 Hz, *J*₂ = 1.6 Hz, 1H), 7.39 (dd, *J*₁ = 8.4 Hz, *J*₂ = 1.2 Hz, 1H), 6.80-6.74 (m, 1H), 6.40 (t, *J=* 4.8 Hz, 1H), 6.22 (s, 2H), 3.99 (s, 2H), 3.79 (s, 3H). ESI-MS: [M + H]⁺ *=* 532.0.

### Preparation of intermediate 2

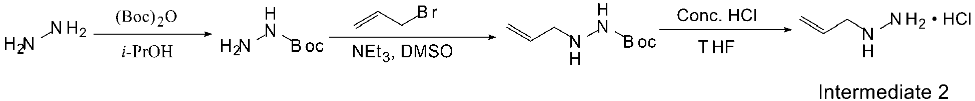

### Step 1: preparation of tert-butyl hydrazinecarboxylate

Hydrazine hydrate (11.3 g) and isopropanol (100 mL) were added into a 250 mL single-necked flask. The mixture was subjected to nitrogen purging and cooled to 0°C, and a solution of di-tert-butyl dicarbonate (17.4 g) in isopropanol (20 mL) was dropwise added to the mixture. After finishing the dropwise addition, the system was allowed to warm to room temperature and stirred to react for 2 hours. The reaction solution was concentrated under reduced pressure to obtain 10.3 g of colorless oily substance.

### Step 2: preparation of tert-butyl 2-allylhydrazine-1-carboxylate

Tert-butyl hydrazinecarboxylate (10.3 g), dimethyl sulfoxide (50 mL), triethylamine (6.5 g) and allyl bromide (7.8 g) were added into a 250 mL single-necked flask. The mixture was subjected to nitrogen purging and stirred at room temperature to react for 3 hours. Water (100 mL) was added to the system. The mixture was extracted with ethyl acetate, and the organic phases were combined, dried and concentrated. The concentrate was purified by silica gel column chromatography to obtain 3.2 g of light yellow oily substance.

### Step 3: preparation of allylhydrazine hydrochloride

Tert-butyl 2-allylhydrazine-1-carboxylate (3.2 g) and tetrahydrofuran (6 mL) were added into a 100 mL single-necked flask, and concentrated hydrochloric acid (6 mL) was slowly dropwise added to the mixture under stirring at room temperature. After finishing the dropwise addition, the mixture was continuously stirred at room temperature to react for 3 hours. The reaction solution was concentrated under reduced pressure to obtain 1.9 g of off-white solid.

### Preparation of intermediate 3

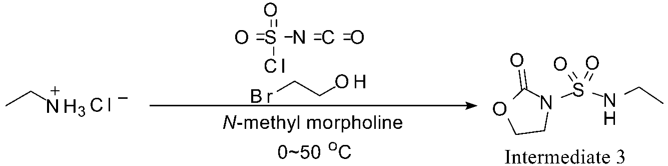

### Preparation of N-ethyl-2-oxooxazoline-3-sulfonamide

In an ice bath, 120 mL of acetonitrile and chlorosulfonyl isocyanate (3.2 g) were added into a 500 mL single-necked flask. Subsequently, 2-bromoethanol (2.8 g) was slowly added to the system. The system was continuously stirred in the ice bath to react for 30 minutes, and then allowed to warm to room temperature and stirred to react for 1 hour. Subsequently, N-methylmorpholine (15.1 g) was slowly added to the system and stirred to react for 10 minutes, and then ethylamine hydrochloride (1.8 g) was slowly added to the system. After finishing the addition, the system was heated to 50°C and stirred to react for 5 hours. Subsequently, the system was gradually cooled to room temperature, and then continuously stirred to react for 15 hours. The mixture was subjected to suction filtration, and the filtrate was concentrated. The concentrate was diluted with ethyl acetate and washed with saturated brine, and the organic phases were dried with anhydrous sodium sulfate, filtered and concentrated to obtain 1.8 g of white solid. ¹H NMR (400 MHz, DMSO-*d*₆, δ): 8.34 (t, *J=* 5.2 Hz, 1H), 4.41-4.37 (m, 2H), 3.96-3.92 (m, 2H), 3.08-3.01 (m, 2H), 1.08 (t, *J =* 7.2 Hz, 3H).

### Preparation of intermediate 4

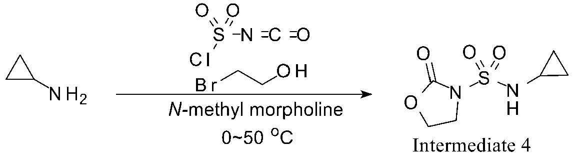

### Preparation of N-cyclopropyl-2-oxooxazoline-3-sulfonamide

In an ice bath, 500 mL of acetonitrile and chlorosulfonyl isocyanate (11.6 g) were added into a 1 L single-necked flask. Subsequently, 2-bromoethanol (10.3 g) was slowly added to the system. The system was continuously stirred in the ice bath to react for 30 minutes, and then allowed to warm to room temperature and stirred to react for 1 hour. Subsequently, N-methylmorpholine (50.0 g) was slowly added to the system and stirred to react for 10 minutes, and then cyclopropylamine (4.7 g) was slowly added to the system. After finishing the addition, the system was heated to 50°C and stirred to react for 5 hours. Subsequently, the system was gradually cooled to room temperature and then continuously stirred for 15 hours. The mixture was subjected to suction filtration, and the filtrate was concentrated to obtain a light orange solid. Most of the solid was dissolved with ethyl acetate and washed with saturated brine, and the organic phases were separated, dried with anhydrous sodium sulfate, filtered and subjected to rotary evaporation to remove the solvent to obtain a crude product. The crude product was slurried with methyl tert-butyl ether to obtain 4.4 g of off-white solid. ¹H NMR (400 MHz, DMSO-*d*₆, δ): 8.72 (s, 1H), 4.44-4.40 (m, 2H), 4.00-3.96 (m, 2H), 2.50-2.45 (m, 1H), 0.65-0.58 (m, 2H), 0.56-0.52 (m, 2H).

### Preparation of intermediate 5

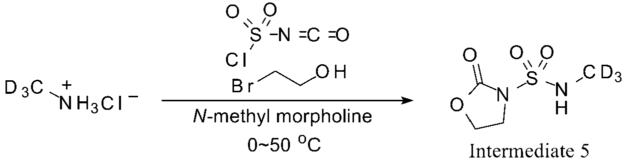

### Preparation of N-methyl-d₃-2-oxooxazolidine-3-sulfonamide

In an ice bath, 720 mL of acetonitrile and chlorosulfonyl isocyanate (20.6 g) were added into a 2 L single-necked flask. Subsequently, 2-bromoethanol (18.2 g) was slowly added to the system. The system was continuously stirred in the ice bath to react for 30 minutes, and then allowed to warm to room temperature and stirred to react for 1 hour. Subsequently, N-methylmorpholine (58.4 g) was slowly added to the system and stirred to react for 10 minutes, and then deuterated methylamine hydrochloride (10.2 g) was slowly added to the system. After finishing the addition, the system was heated to 50°C and stirred to react for 5 hours. Subsequently, the system was gradually cooled to room temperature and then continuously stirred to react for 15 hours. The mixture was subjected to suction filtration, and the filtrate was concentrated. The concentrate was diluted with water and extracted with ethyl acetate, and the organic phases were combined, washed with saturated brine and dried with anhydrous sodium sulfate. The mixture was filtered and subjected to rotary evaporation to remove the solvent to obtain 11.9 g of light yellow solid. ¹H NMR (400 MHz, DMSO-*d*₆, δ): 8.17 (s, 1H), 4.42-4.38 (m, 2H), 3.96-3.93 (m, 2H).

### Preparation of compounds

### Example 1

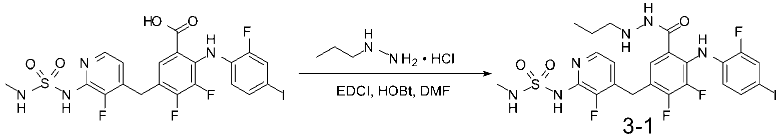

### Preparation of N'-propyl-3,4-difluoro-5-((3-fluoro-2-((N-methylsulfamoyl)amino)pyridin-4-yl)methyl)-2-((2-fluoro-4-iodophenyl)amino)benzohydrazide

3,4-difluoro-5-((3-fluoro-2-((N-methylsulfamoyl)amino)pyridin-4-yl)methyl)-2-((2-fl uoro-4-iodophenyl)amino)benzoic acid (500 mg), N,N-dimethylformamide (10 mL), 1-hydroxybenzotriazole (170 mg) and 1-(3-dimethylaminopropyl)-3-ethylcarbodiimide hydrochloride (470 mg) were added into a 100 mL single-necked flask, and the mixture was stirred at room temperature to react for 1 hour. Propylhydrazine hydrochloride (170 mg) and N,N-diisopropylethylamine (320 mg) were added to the system, and continuously stirred at room temperature to react for 1.5 hours. The reaction solution was slowly added to a saturated sodium bicarbonate solution and extracted with ethyl acetate, and the organic phases were combined, dried and concentrated. The concentrate was purified by silica gel column chromatography to obtain 190 mg of off-white solid. ESI-MS: [M + H]⁺ = 667.1.

### Example 2

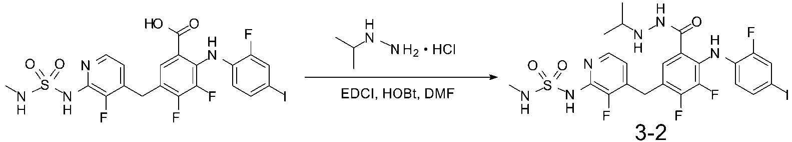

### Preparation of N'-isopropyl-3,4-difluoro-5-((3-fluoro-2-((N-methylsulfamoyl)amino)pyridin-4-yl)methyl) -2-((2-fluoro-4-iodophenyl)amino)benzohydrazide

3,4-difluoro-5-((3-fluoro-2-((N-methylsulfamoyl)amino)pyridin-4-yl)methyl)-2-((2-fl uoro-4-iodophenyl)amino)benzoic acid (400 mg), N,N-dimethylformamide (20 mL), 1-hydroxybenzotriazole (133 mg) and 1-(3-dimethylaminopropyl)-3-ethylcarbodiimide hydrochloride (188 mg) were added into a 100 mL single-necked flask, and the mixture was stirred at room temperature to react for 1 hour. Isopropylhydrazine hydrochloride (145 mg) and N,N-diisopropylethylamine (338 mg) were added to the system, and continuously stirred at room temperature to react for 2 hours. The reaction solution was slowly added to a saturated sodium bicarbonate solution and extracted with ethyl acetate, and the organic phases were combined, dried and concentrated. The concentrate was purified by silica gel column chromatography to obtain 130 mg of off-white solid. ¹H NMR (400 MHz, DMSO-*d*₆): δ 10.42 (s, 1H), 10.12 (d, *J=* 6.4 Hz, 1H), 8.83 (s, 1H), 8.02 (d, *J=* 5.2 Hz, 1H), 7.58 (dd, *J=* 10.8, 2.0 Hz, 1H), 7.48 (d, *J= 7.2* Hz, 1H), 7.35 (d, *J =* 8.4 Hz, 1H), 7.07-7.00 (m, 1H), 6.96 (t, *J* = 4.8 Hz, 1H), 6.69-6.63 (m, 1H), 4.99-4.97 (m, 1H), 4.08 (s, 2H), 3.01-2.94 (m, 1H), 2.52-2.51 (m, 3H), 0.96 (d, *J=* 6.0 Hz, 6H). ESI-MS: [M + H]⁺ = 667.1.

### Example 3

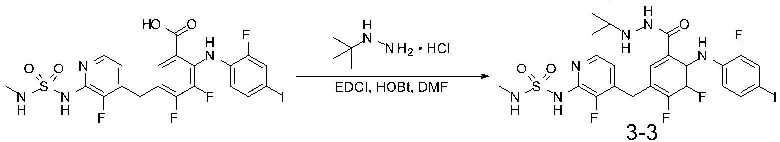

### Preparation of N'-tert-butyl-3,4-difluoro-5-((3-fluoro-2-((N-methylsulfamoyl)amino)pyridin-4-yl)methyl) -2-((2-fluoro-4-iodophenyl)amino)benzohydrazide

3,4-difluoro-5-((3-fluoro-2-((N-methylsulfamoyl)amino)pyridin-4-yl)methyl)-2-((2-fl uoro-4-iodophenyl)amino)benzoic acid (500 mg), N,N-dimethylformamide (15 mL), 1-hydroxybenzotriazole (170 mg) and 1-(3-dimethylaminopropyl)-3-ethylcarbodiimide hydrochloride (470 mg) were added into a 100 mL single-necked flask, and the mixture was stirred at room temperature to react for 1 hour. Tert-butylhydrazine hydrochloride (200 mg) and N,N-diisopropylethylamine (320 mg) were added to the system, and continuously stirred at room temperature to react for 2 hours. The reaction solution was slowly added to a saturated sodium bicarbonate solution and extracted with ethyl acetate, and the organic phases were combined, dried and concentrated. The concentrate was purified by silica gel column chromatography to obtain 180 mg of off-white solid. ¹H NMR (400 MHz, DMSO-*d*₆): δ 10.43 (s, 1H), 9.99 (d, *J=* 6.8 Hz, 1H), 8.67 (s, 1H), 8.02 (d, *J=* 4.8 Hz, 1H), 7.58 (dd, *J=* 10.8, 2.0 Hz, 1H), 7.51 (d, *J=* 6.8 Hz, 1H), 7.34 (d, *J=* 8.4 Hz, 1H), 7.06-7.02 (m, 1H), 6.96 (t, *J=* 4.4 Hz, 1H), 6.67-6.60 (m, 1H), 4.92 (d, *J =* 7.2 Hz, 1H), 4.10 (s, 2H), 2.52-2.51 (m, 3H), 1.00 (s, 9H). ESI-MS: [M + H]⁺ = 681.1.

### Example 4

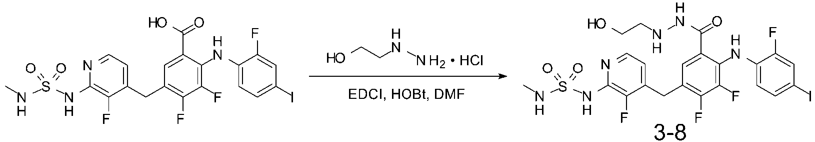

### Preparation of N'-(hydroxyethyl)-3,4-difluoro-5-((3-fluoro-2-((N-methylsulfamoyl)amino)pyridin-4-yl)m ethyl)-2-((2-fluoro-4-iodophenyl)amino)benzohydrazide

3,4-difluoro-5-((3-fluoro-2-((N-methylsulfamoyl)amino)pyridin-4-yl)methyl)-2-((2-fl uoro-4-iodophenyl)amino)benzoic acid (1.0 g), N,N-dimethylformamide (20 mL), 1-hydroxybenzotriazole (266 mg) and 1-(3-dimethylaminopropyl)-3-ethylcarbodiimide hydrochloride (629 mg) were added into a 100 mL single-necked flask, and the mixture was stirred at room temperature to react for 1 hour. hydroxyethylhydrazine hydrochloride (374 mg) and N,N-diisopropylethylamine (423 mg) were added to the system, and continuously stirred at room temperature to react for 2 hours. The reaction solution was slowly added to a saturated sodium bicarbonate solution and extracted with ethyl acetate, and the organic phases were combined, dried and concentrated. The concentrate was purified by silica gel column chromatography to obtain 350 mg of off-white solid. ESI-MS: [M + H]⁺ = 669.0.

### Example 5

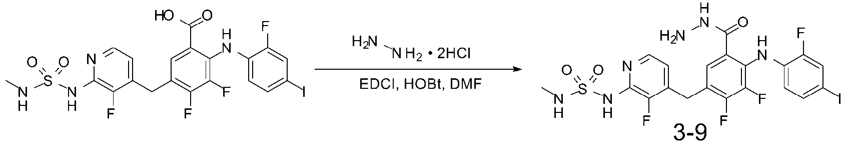

### Preparation of 3,4-difluoro-5-((3-fluoro-2-((N-methylsulfamoyl)amino)pyridin-4-yl)methyl)-2-((2-fluoro-4-iodophenyl)amino)benzohydrazide

3,4-difluoro-5-((3-fluoro-2-((N-methylsulfamoyl)amino)pyridin-4-yl)methyl)-2-((2-fi uoro-4-iodophenyl)amino)benzoic acid (400 mg), N,N-dimethylformamide (20 mL), 1-hydroxybenzotriazole (133 mg) and 1-(3-dimethylaminopropyl)-3-ethylcarbodiimide hydrochloride (188 mg) were added into a 100 mL single-necked flask, and the mixture was stirred at room temperature to react for 1 hour. Hydrazine dihydrochloride (276 mg) and N,N-diisopropylethylamine (680 mg) were added to the system, and continuously stirred at room temperature to react for 2 hours. The reaction solution was slowly added to a saturated sodium bicarbonate solution and extracted with ethyl acetate, and the organic phases were combined, dried and concentrated. The concentrate was purified by silica gel column chromatography to obtain 100 mg of light yellow solid. ESI-MS: [M + H]⁺ = 625.0.

### Example 6

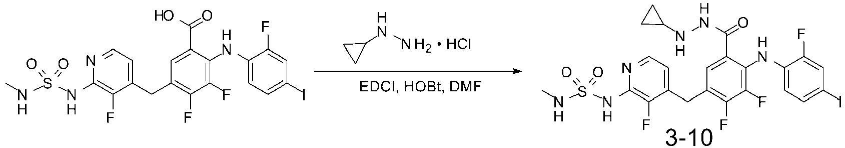

### Preparation of N'-cyclopropyl-3,4-difluoro-5-((3-fluoro-2-((N-methylsulfamoyl)amino)pyridin-4-yl)meth yl)-2-((2-fluoro-4-iodophenyl)amino)benzohydrazide

3,4-difluoro-5-((3-fluoro-2-((N-methylsulfamoyl)amino)pyridin-4-yl)methyl)-2-((2-fl uoro-4-iodophenyl)amino)benzoic acid (700 mg), N,N-dimethylformamide (15 mL), 1-hydroxybenzotriazole (187 mg) and 1-(3-dimethylaminopropyl)-3-ethylcarbodiimide hydrochloride (441 mg) were added into a 100 mL single-necked flask, and the mixture was stirred at room temperature to react for 1 hour. Cyclopropylhydrazine hydrochloride (375 mg) and N,N-diisopropylethylamine (445 mg) were added to the system, and continuously stirred at room temperature to react for 2 hours. The reaction solution was slowly added to a saturated sodium bicarbonate solution and extracted with ethyl acetate, and the organic phases were combined, dried and concentrated. The concentrate was purified by silica gel column chromatography to obtain 350 mg of off-white solid. ¹H NMR (400 MHz, DMSO): δ 10.43 (s, 1H), 10.20 (d, *J =* 6.0 Hz, 1H), 8.95 (s, 1H), 8.02 (d, *J= 4.4* Hz, 1H), 7.59 (dd, *J* = 10.8, 2.0 Hz, 1H), 7.49 (d, *J =* 6.8 Hz, 1H), 7.36 (d, *J =* 8.4 Hz, 1H), 7.04 (d, *J =* 4.4 Hz, 1H), 6.95 (brs, 1H), 6.71-6.66 (m, 1H), 5.67-5.64 (m, 1H), 4.07 (s, 2H), 2.52-2.50 (m, 4H), 0.39-0.38 (m, 4H). ESI-MS: [M + H]⁺ = 665.1.

### Example 7

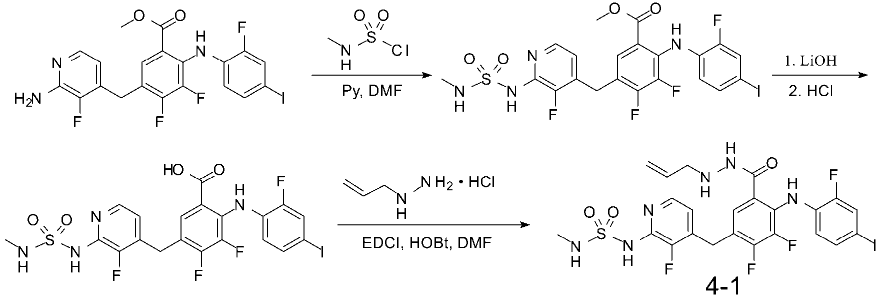

### Step 1: preparation of methyl 3,4-difluoro-5-((3-fluoro-2-((N-methylsulfamoyl)amino)pyridin-4-yl)methyl)-2-((2-fluoro-4-iodophenyl)amino)benzoate

Methyl 5-((2-amino-3-fluoropyridin-4-yl)methyl)-3,4-difluoro-2-((2-fluoro-4-iodophenyl)amino)b enzoate (460 mg), N,N-dimethylformamide (10 mL) and pyridine (680 mg) were added into a 100 mL single-necked flask, and the mixture was cooled to -20°C. Methylsulfamoyl chloride (560 mg) was slowly dropwise added to the system, and after finishing the dropwise addition, the mixture was allowed to react for 30 minutes. The reaction solution was poured into water and stirred at room temperature for 10 minutes. The mixture was subjected to suction filtration, the filter cake was dissolved with ethyl acetate, and the organic phases were washed with saturated brine, dried and concentrated to obtain 531 mg of off-white solid. ESI-MS: [M + H]⁺ = 625.0.

### Step 2: preparation of 3,4-difluoro-5-((3-fluoro-2-((N-methylsulfamoyl)amino)pyridin-4-yl)methyl)-2-((2-fluoro-4-iodophenyl)amino)benzoic acid

The product (400 mg) from the previous step and tetrahydrofuran (6 mL) were added into a 100 mL single-necked flask, and the mixture was stirred at room temperature until a clear solution was obtained. An aqueous solution (3 mL) of lithium hydroxide monohydrate (81 mg) was dropwise added to the system, and after finishing the dropwise addition, the mixture was continuously stirred at room temperature to react for 3 hours. The mixture was concentrated under reduced pressure to remove tetrahydrofuran, water was added to the system, 1 M dilute hydrochloric acid was added to adjust the pH value of the system to be 2-3, and the mixture was stirred for 10 minutes. The mixture was filtered, and the filter cake was collected, washed with water and dried to obtain 330 mg of off-white solid. ESI-MS: [M + H]⁺= 611.0.

### Step 3: preparation of N'-allyl-3,4-difluoro-5-((3-fluoro-2-((N-methylsulfamoyl)amino)pyridin-4-yl)methyl)-2-(( 2-fluoro-4-iodophenyl)amino)benzohydrazide

The product (700 mg) from the previous step and N,N-dimethylformamide (20 mL) were added into a 100 mL single-necked flask, and the mixture was cooled to 0°C in an ice bath. Subsequently, 1-hydroxybenzotriazole (230 mg) and 1-(3-dimethylaminopropyl)-3-ethylcarbodiimide hydrochloride (660 mg) were added to the mixture, the thus obtained mixture was stirred to react for 5 minutes, and then allowed to warm to room temperature and react for 2 hours. Allylhydrazine hydrochloride (250 mg) and N,N-diisopropylethylamine (590 mg) were added to the system, and after finishing the addition, the mixture was continuously stirred at room temperature to react for 2 hours. The reaction solution was slowly added to a saturated sodium bicarbonate solution and extracted with ethyl acetate, and the organic phases were combined, dried and concentrated. The concentrate was purified by preparative HPLC to obtain 200 mg of white solid. ¹H NMR (400 MHz, DMSO-*d*₆): δ 10.42 (s, 1H), 10.11 (d, *J =* 6.3 Hz, 1H), 8.81 (s, 1H), 8.02 (d, *J =* 4.9 Hz, 1H), 7.58 (dd, *J =* 10.8, 1.8 Hz, 1H), 7.40 (d, *J =* 6.9 Hz, 1H), 7.35 (d, *J =* 8.5 Hz, 1H), 7.04-6.95 (m, 2H), 6.68-6.62 (m, 1H), 5.85-5.75 (m, 1H), 5.32-5.28 (m, 1H), 5.10 (d, *J= 17.3* Hz, 1H), 5.02 (d, *J =* 10.2 Hz, 1H), 4.06 (s, 2H), 3.34 (t, *J =* 5.5 Hz, 2H), 2.51-2.50 (m, 3H). ESI-MS: [M + H]⁺ = 665.1.

### Example 8

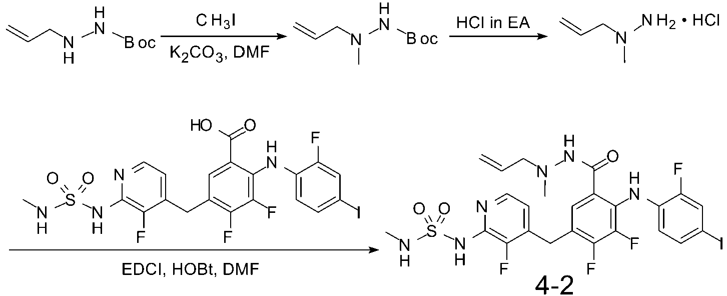

### Step 1: preparation of tert-butyl 2-allyl-2-methylhydrazine-1-carboxylate

Tert-butyl 2-allylhydrazine-1-carboxylate (800 mg), potassium carbonate (1.3 g), iodomethane (979 mg) and N,N-dimethylformamide (5 mL) were added into a 50 mL single-necked flask, and the mixture was heated to 30°C and stirred to react for 4.5 hours. The reaction solution was poured into water and extracted with ethyl acetate, and the organic phases were combined, dried and concentrated to obtain 800 mg of light yellow-green liquid. ESI-MS: [M + H]⁺= 187.1.

### Step 2: preparation of 1-allyl-1-methylhydrazine hydrochloride

The product (800 mg) from the previous step and a solution of hydrogen chloride in ethyl acetate (4 M, 5 mL) were added into a 50 mL single-necked flask, and the mixture was stirred at room temperature to react for 2 hours. The reaction solution was concentrated to obtain 500 mg of yellow viscous substance. ESI-MS: [M + H]⁺= 87.1.

### Step 3: preparation of N'-methyl-N'-allyl-3,4-difluoro-5-((3-fluoro-2-((N-methylsulfamoyl)amino)pyridin-4-yl)methyl)-2-((2-fluoro-4-iodophenyl)amino)benzohydrazide

3,4-difluoro-5-((3-fluoro-2-((N-methylsulfamoyl)amino)pyridin-4-yl)methyl)-2-((2-fi uoro-4-iodophenyl)amino)benzoic acid (400 mg) and N,N-dimethylformamide (8 mL) were added into a 100 mL single-necked flask, and the mixture was cooled to 0°C in an ice bath. Subsequently, 1-hydroxybenzotriazole (130 mg) and 1-(3-dimethylaminopropyl)-3-ethylcarbodiimide hydrochloride (380 mg) were added to the mixture, stirred to react for 5 minutes, and then allowed to warm to room temperature and react for 2 hours. 1-allyl-1-methylhydrazine hydrochloride (500 mg) and N,N-diisopropylethylamine (250 mg) were added to the system, and after finishing the addition, the mixture was continuously stirred at room temperature to react for 2 hours. The reaction solution was slowly added to a saturated sodium bicarbonate solution and extracted with ethyl acetate, and the organic phases were combined, dried and concentrated. The concentrate was purified by silica gel column chromatography to obtain 88 mg of off-white solid. ¹H NMR (400 MHz, DMSO-*d*₆): δ 10.43 (s, 1H), 9.48 (s, 1H), 8.54 (s, 1H), 8.02 (d, *J=* 4.0 Hz, 1H), 7.58 (dd, *J =* 10.8, 2.0 Hz, 1H), 7.35-7.33 (m, 2H), 7.04 (brs, 1H), 6.93 (brs, 1H), 6.65-6.60 (m, 1H), 5.81-5.71 (m, 1H), 5.10 (dd, *J =* 17.2, 1.6 Hz, 1H), 4.98 (dd, *J=* 10.4, 1.6 Hz, 1H), 4.07 (s, 2H), 3.34-3.33 (m, 2H), 2.51-2.50 (m, 6H). ESI-MS: [M + H]⁺ = 679.1.

### Example 9

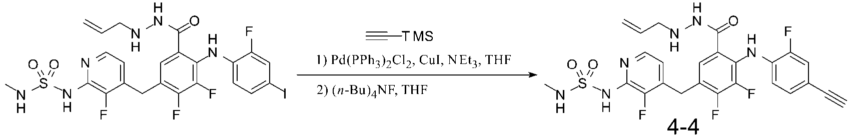

### Preparation of N'-allyl-3,4-difluoro-5-((3-fluoro-2-((N-methylsulfamoyl)amino)pyridin-4-yl)methyl)-2-(( 2-fluoro-4-ethynylphenyl)amino)benzohydrazide

N'-allyl-3,4-difluoro-5-((3-fluoro-2-((N-methylsulfamoyl)amino)pyridin-4-yl)methyl) -2-((2-fluoro-4-iodophenyl)amino)benzohydrazide (150 mg), copper(I) iodide (1 mg), triethylamine (0.5 mL), tetrahydrofuran (5 mL), trimethylsilylacetylene (100 mg) and bis(triphenylphosphine)palladium(II) dichloride (8 mg) were added into a 100 mL single-necked flask, and the mixture was subjected to nitrogen purging and stirred at room temperature to react for 1 hour. Subsequently, a solution of tetrabutylammonium fluoride in tetrahydrofuran (1 M, 3 mL) was added to the system, and continuously stirred at room temperature to react for 1 hour. The mixture was filtered, the filter cake was washed with ethyl acetate, and the organic phases were combined, washed with water, dried and concentrated. The concentrate was purified by silica gel column chromatography to obtain a product. ESI-MS: [M + H]⁺ = 563.2.

### Example 10

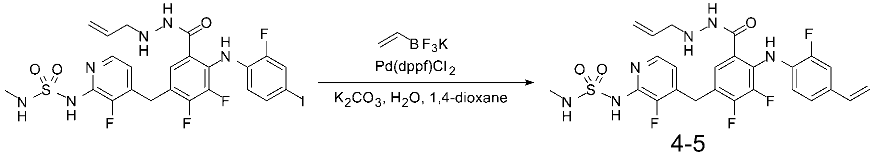

### Preparation of N'-allyl-3,4-difluoro-5-((3-fluoro-2-((N-methylsulfamoyl)amino)pyridin-4-yl)methyl)-2-(( 2-fluoro-4-vinylphenyl)amino)benzohydrazide

N'-allyl-3,4-difluoro-5-((3-fluoro-2-((N-methylsulfamoyl)amino)pyridin-4-yl)methyl) -2-((2-fluoro-4-iodophenyl)amino)benzohydrazide (150 mg), potassium vinyltrifluoroborate (61 mg), potassium carbonate (94 mg), 1,4-dioxane (5 mL), water (1.5 mL) and [1,1'-bis(diphenylphosphino)ferrocene]dichloropalladium(II) (8 mg) were added into a 50 mL single-necked flask, and the mixture was subjected to nitrogen purging, and heated to 95°C and stirred to react for 1 hour. After cooling to room temperature, the reaction solution was poured into water for quenching, the aqueous phase was extracted with ethyl acetate, and the organic phases were combined, dried and concentrated. The concentrate was purified by silica gel column chromatography to obtain a product. ESI-MS: [M + H]⁺ = 565.2.

### Example 11

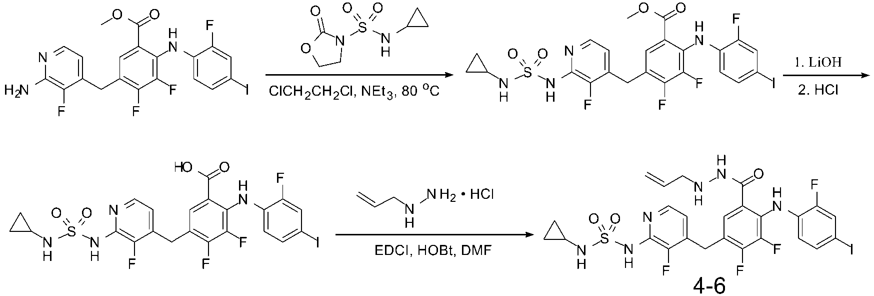

### Step 1 : preparation of methyl 5-((2-((N-cyclopropylsulfamoyl)amino)-3-fluoropyridin-4-yl)methyl)-3,4-difluoro-2-((2-fl uoro-4-iodophenyl)amino)benzoate

N-cyclopropyl-2-oxooxazolidine-3-sulfonamide (970 mg), methyl 5-((2-amino-3-fluoropyridin-4-yl)methyl)-3,4-difluoro-2-((2-fluoro-4-iodophenyl)amino)b enzoate (500 mg), triethylamine (570 mg) and 1,2-dichloroethane (25 mL) were added into a 100 mL single-necked flask, and the mixture was heated to 80°C and allowed to react for 4 hours. After cooling to room temperature, the reaction solution was poured into a water/tetrahydrofuran mixed solvent (90/10, v/v), the mixture was extracted with ethyl acetate, and the organic phases were combined, dried and concentrated to obtain 600 mg of brown solid. ESI-MS: [M + H]⁺ = 651.0.

### Step 2: preparation of 5-((2-((N-cyclopropylsulfamoyl)amino)-3-fluoropyridin-4-yl)methyl)-3,4-difluoro-2-((2-fl uoro-4-iodophenyl)amino)benzoic acid

The product (600 mg) from the previous step, tetrahydrofuran (30 mL) and water (12 mL) were sequentially added into a 50 mL single-necked flask, and the mixture was stirred until a clear solution was obtained. Lithium hydroxide monohydrate (193 mg) was added to the system, and after finishing the addition, the mixture was stirred at room temperature to react for 1.5 hours. An aqueous hydrochloric acid solution (1 M) was added to adjust the pH value of the system to be 4-5, and the mixture was concentrated under reduced pressure to remove tetrahydrofuran. Water was added to the concentrate, the mixture was filtered, and a filter cake was collected to obtain 580 mg of brown solid. ESI-MS: [M + H]⁺ = 637.0.

### Step 3: preparation of N'-allyl-3,4-difluoro-5-((3-fluoro-2-((N-cyclopropylsulfamoyl)amino)pyridin-4-yl)methyl) -2-((2-fluoro-4-iodophenyl)amino)benzohydrazide

The product (580 mg) from the previous step, N,N-dimethylformamide (20 mL), 1-hydroxybenzotriazole (216 mg) and 1-(3-dimethylaminopropyl)-3-ethylcarbodiimide hydrochloride (305 mg) were added into a 100 mL single-necked flask, and the mixture was stirred at room temperature to react for 1 hour. Allylhydrazine hydrochloride (265 mg) and N,N-diisopropylethylamine (793 mg) were added to the system, and the mixture was continuously stirred at room temperature to react for 30 minutes. The reaction solution was slowly added to a saturated aqueous sodium bicarbonate solution, the mixture was extracted with ethyl acetate, and the organic phases were combined, dried and concentrated. The concentrate was purified by silica gel column chromatography to obtain a product. ESI-MS: [M + H]⁺ = 691.1.

### Example 12

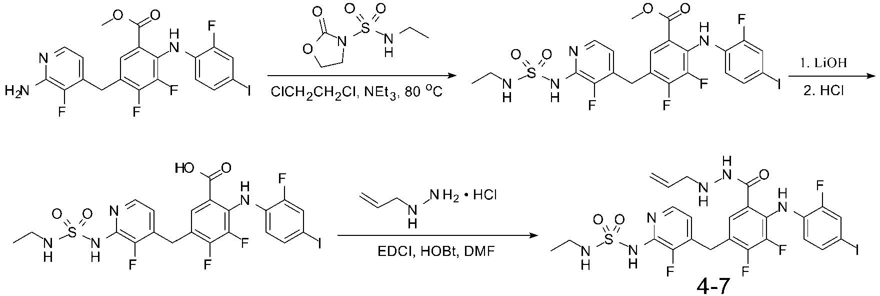

### Step 1 : preparation of methyl 5-((2-((N-ethylsulfamoyl)amino)-3-fluoropyridin-4-yl)methyl)-3,4-difluoro-2-((2-fluoro-4-iodophenyl)amino)benzoate

N-ethyl-2-oxooxazolidine-3-sulfonamide (5.5 g), methyl 5-((2-amino-3-fluoropyridin-4-yl)methyl)-3,4-difluoro-2-((2-fluoro-4-iodophenyl)amino)b enzoate (1.5 g), triethylamine (3.1 g) and 1,2-dichloroethane (50 mL) were added into a 100 mL single-necked flask, and the mixture was heated to 80°C and allowed to react for 4.5 hours. After cooling to room temperature, the system was poured into a water/tetrahydrofuran mixed solvent (90/10, v/v), the aqueous phase was extracted with ethyl acetate, and the organic phases were combined, dried and concentrated to obtain 1.0 g of yellow oily substance. ESI-MS: [M + H]⁺ = 639.0.

### Step 2: preparation of 5-((2-((N-ethylsulfamoyl)amino)-3-fluoropyridin-4-yl)methyl)-3,4-difluoro-2-((2-fluoro-4-iodophenyl)amino)benzoic acid

The product (1.0 g) from the previous step, tetrahydrofuran (20 mL) and water (5 mL) were sequentially added into a 50 mL single-necked flask, and the mixture was stirred until a clear solution was obtained. Subsequently, lithium hydroxide monohydrate (130 mg) was added to the system, and after the addition, the mixture was continuously stirred at room temperature to react for 3 hours. An aqueous hydrochloric acid solution (1 M) was added to the reaction solution to adjust the pH value of the system to be 5-6, and mixture was concentrated under reduced pressure to remove tetrahydrofuran. Subsequently, the concentrate was diluted with water and filtered, and a filter cake was collected to obtain 750 mg of brown solid. ESI-MS: [M - H]⁻ = 623.0.

### Step 3: preparation of N'-allyl-3,4-difluoro-5-((3-fluoro-2-((N-ethylsulfamoyl)amino)pyridin-4-yl)methyl)-2-((2-fluoro-4-iodophenyl)amino)benzohydrazide

The product (750 mg) from the previous step, N,N-dimethylformamide (8 mL), 1-hydroxybenzotriazole (240 mg) and 1-(3-dimethylaminopropyl)-3-ethylcarbodiimide hydrochloride (690 mg) were added into a 100 mL single-necked flask, and the mixture was stirred at room temperature to react for 2 hours. Allylhydrazine hydrochloride (260 mg) and N,N-diisopropylethylamine (470 mg) were added to the system, and continuously stirred at room temperature to react for 1 hour. The reaction solution was slowly added to a saturated aqueous sodium bicarbonate solution, the mixture was extracted with ethyl acetate, and the organic phases were combined, dried and concentrated. The concentrate was purified by silica gel column chromatography to obtain a product. ESI-MS: [M + H]⁺ = 679.1.

### Example 13

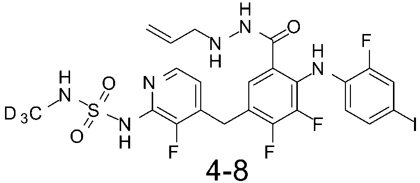

With reference to Example 12, compound 4-8 was synthesized by replacing N-ethyl-2-oxooxazolidine-3-sulfonamide with N-methyl-*d*₃-2-oxooxazolidine-3-sulfonamide. ESI-MS: [M + H]⁺ = 668.1.

### Example 14

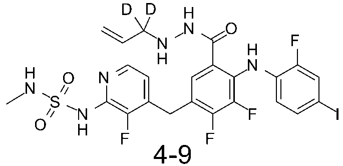

With reference to the synthesis method of Intermediate 2, (allyl-1,1-*d*₂)hydrazine hydrochloride was synthesized, and then compound 4-9 was synthesized according to Example 7. ESI-MS: [M + H]⁺ = 667.1.

### Example 15

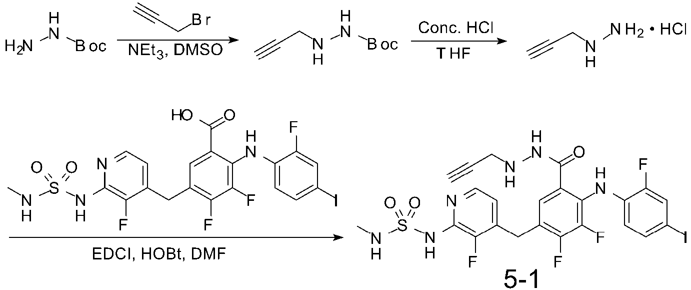

### Step 1: preparation of tert-butyl 2-(prop-2-yn-1-yl)hydrazine-1-carboxylate

Tert-butyl hydrazinecarboxylate (10.0 g), dimethyl sulfoxide (50 mL), triethylamine (6.8 g) and propargyl bromide (8.0 g) were added into a 250 mL single-necked flask, and the mixture was subjected to nitrogen purging and then stirred at room temperature to react for 3 hours. Water was added to the reaction solution, the mixture was extracted with ethyl acetate, and the organic phases were combined, dried and concentrated. The concentrate was purified by silica gel column chromatography to obtain 1.5 g of white solid.

### Step 2: preparation of prop-2-yn-1-ylhydrazine hydrochloride

The product (2.2 g) from the previous step and tetrahydrofuran (6 mL) were added into a 100 mL single-necked flask, and concentrated hydrochloric acid (6 mL) was slowly dropwise added to the mixture under stirring at room temperature. After finishing the dropwise addition, the system was continuously stirred at room temperature to react for 3 hours. The reaction solution was concentrated to obtain 1.0 g of yellow oily substance. ESI-MS: [M + H]⁺ = 71.1.

### Step 3: preparation of N'-(prop-2-yn-1-yl)-3,4-difluoro-5-((3-fluoro-2-((N-methylsulfamoyl)amino)pyridin-4-yl) methyl)-2-((2-fluoro-4-iodophenyl)amino)benzohydrazide

3,4-difluoro-5-((3-fluoro-2-((N-methylsulfamoyl)amino)pyridin-4-yl)methyl)-2-((2-fl uoro-4-iodophenyl)amino)benzoic acid (1.0 g) and N,N-dimethylformamide (20 mL) were added into a 100 mL single-necked flask, and the mixture was cooled to 0°C in an ice bath. Subsequently, 1-hydroxybenzotriazole (266 mg) and 1-(3-dimethylaminopropyl)-3-ethylcarbodiimide hydrochloride (629 mg) were added, and after stirring to react for 5 minutes, the mixture was allowed to warm to room temperature and react for 2 hours. Prop-2-yn-1-ylhydrazine hydrochloride (351 mg) and N,N-diisopropylethylamine (793 mg) were added to the system, and continuously stirred at room temperature to react for 2 hours. The reaction solution was slowly added to a saturated sodium bicarbonate solution and extracted with ethyl acetate, and the organic phases were combined, dried and concentrated. The concentrate was purified by silica gel column chromatography to obtain 200 mg of off-white solid. ESI-MS: [M + H]⁺ = 663.0.

### Example 16

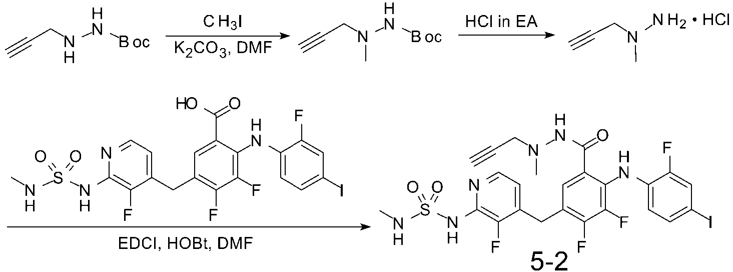

### Step 1: preparation of tert-butyl 2-methyl-2-(prop-2-yn-1-yl)hydrazine-1-carboxylate

Tert-butyl 2-(prop-2-yn-1-yl)hydrazine-1-carboxylate (1.4 g), potassium carbonate (1.7 g), iodomethane (1.8 g) and N,N-dimethylformamide (20 mL) were added into a 50 mL single-necked flask, and the mixture was heated to 30°C and stirred to react for 2 hours. The reaction solution was poured into water and extracted with ethyl acetate, and the organic phases were combined, dried and concentrated to obtain 1.0 g of colorless liquid.

### Step 2: preparation of 1-methyl-1-(prop-2-yn-1-yl)hydrazine hydrochloride

The product (1.0 g) from the previous step and a solution of hydrogen chloride in ethyl acetate (4 M, 10 mL) were added into a 50 mL single-necked flask, and the mixture was stirred at room temperature to react for 2 hours. The reaction solution was concentrated to obtain 410 mg of white solid. ESI-MS: [M + H]⁺= 85.1.

### Step 3: preparation of N'-methyl-N'-(prop-2-yn-1-yl)-3,4-difluoro-5-((3-fluoro-2-((N-methylsulfamoyl)amino)pyr idin-4-yl)methyl)-2-((2-fluoro-4-iodophenyl)amino)benzohydrazide

3,4-difluoro-5-((3-fluoro-2-((N-methylsulfamoyl)amino)pyridin-4-yl)methyl)-2-((2-fl uoro-4-iodophenyl)amino)benzoic acid (400 mg) and N,N-dimethylformamide (10 mL) were added into a 100 mL single-necked flask, and the mixture was cooled to 0°C in an ice bath. Subsequently, 1-hydroxybenzotriazole (107 mg) and 1-(3-dimethylaminopropyl)-3-ethylcarbodiimide hydrochloride (252 mg) were added to the system, and stirred to react for 5 minutes, and then the mixture was allowed to warm to room temperature and react for 2 hours. 1-methyl-1-(prop-2-yn-1-yl)hydrazine hydrochloride (95 mg) and N,N-diisopropylethylamine (254 mg) were added to the system, and continuously stirred at room temperature to react for 2 hours. The reaction solution was slowly added to a saturated sodium bicarbonate solution and extracted with ethyl acetate, and the organic phases were combined, dried and concentrated. The concentrate was purified by silica gel column chromatography to obtain 150 mg of off-white solid. ¹H NMR (400 MHz, DMSO-*d*₆): δ 10.43 (s, 1H), 9.62 (s, 1H), 8.55 (s, 1H), 8.02 (d, *J =* 4.4 Hz, 1H), 7.57 (dd, *J =* 10.8, 2.0 Hz, 1H), 7.41 (d, *J =* 7.2 Hz, 1H), 7.35 (dd, *J =* 8.4, 0.8 Hz, 1H), 7.04 (brs, 1H), 6.93 (brs, 1H), 6.67-6.61 (m, 1H), 4.07 (s, 2H), 3.56 (d, *J =* 2.0 Hz, 2H), 3.14 (t, *J =* 2.0 Hz, 1H), 2.54-2.51 (m, 6H). ESI-MS: [M + H]⁺ = 677.1.

### Example 17

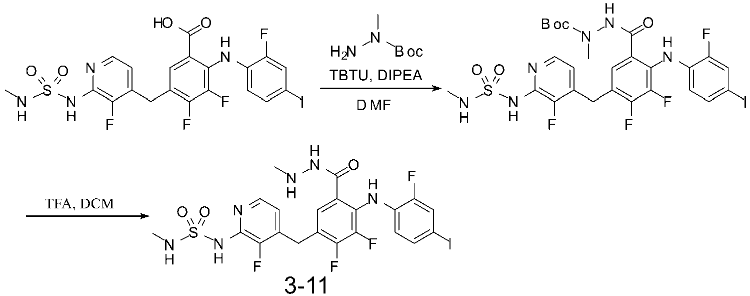

### Step 1 : preparation of tert-butyl 2-(3,4-difluoro-5-((3-fluoro-2-((N-methylsulfamoyl)amino)pyridin-4-yl)methyl)-2-((2-fluo ro-4-iodophenyl)amino)benzoyl)-1-methylhydrazine-1-carboxylate

3,4-difluoro-5-((3-fluoro-2-((N-methylsulfamoyl)amino)pyridin-4-yl)methyl)-2-((2-fl uoro-4-iodophenyl)amino)benzoic acid (500 mg), 1-(tert-butoxycarbonyl)-1-methylhydrazine (145 mg) and N,N-dimethylformamide (10 mL) were added into a reaction flask, and the mixture was cooled to 0°C in an ice bath. Subsequently, O-(benzotriazol-1-yl)-N,N,N',N'-tetramethyluronium tetrafluoroborate (395 mg) and N,N-diisopropylethylamine (265 mg) were added to the system, and after finishing the addition, the mixture was allowed to warm to room temperature and stirred to react for 2 hours. The reaction solution was slowly added to a saturated sodium bicarbonate solution, the mixture was extracted with ethyl acetate, and the organic phases were combined, dried and concentrated. The concentrate was purified by silica gel column chromatography to obtain 442 mg of product.

### Step 2: preparation of N'-methyl-3,4-difluoro-5-((3-fluoro-2-((N-methylsulfamoyl)amino)pyridin-4-yl)methyl)-2-((2-fluoro-4-iodophenyl)amino)benzohydrazide

The product (440 mg) from the previous step, dichloromethane (10 mL) and trifluoroacetic acid (2 mL) were added into a reaction flask, and after finishing the addition, the mixture was stirred at room temperature for 2 hours. The reaction mixture was slowly added to a cold aqueous sodium bicarbonate solution, the mixture was extracted with dichloromethane, and organic phases were collected. The organic phases were washed with saturated aqueous sodium chloride solution, dried with anhydrous sodium sulfate, filtered and concentrated. The concentrate was purified by silica gel column chromatography to obtain 263 mg of product. ESI-MS: [M + H]⁺ = 639.0.

### Example 18

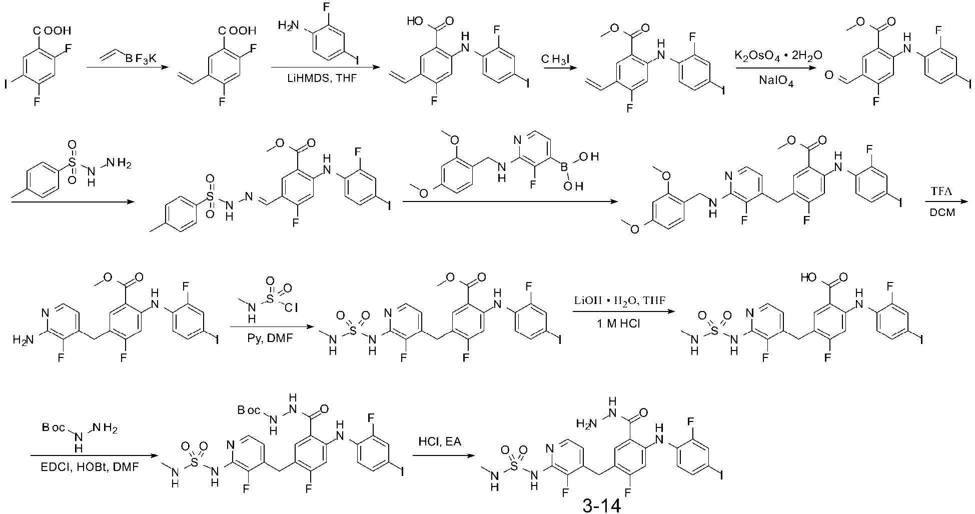

### Step 1: preparation of 2,4-difluoro-5-vinylbenzoic acid

2,4-difluoro-5-iodobenzoic acid (10.0 g), potassium vinyltrifluoroborate (5.7 g), potassium phosphate (11.2 g), methanol (200 mL), water (100 mL) and [1,1'-bis(diphenylphosphino)ferrocene]dichloropalladium(II) (2.6 g) were sequentially added into a 3 L three-necked flask. The system was subjected to nitrogen purging, and heated to 60°C and allowed to react for 21 hours. After cooling to room temperature, the system was filtered through diatomaceous earth, the filtrate was collected and concentrated, and 1 M HCl was added to adjust the pH value of the system to be 4-5. Subsequently, the system was extracted with methyl tert-butyl ether, the organic phases were combined, washed with saturated brine, dried and concentrated. The concentrate was slurried with a petroleum ether/dichloromethane mixed solvent (v/v = 9/1) to obtain 6.3 g of brown solid. ESI-MS: [M-H]⁻= 183.0.

### Step 2: preparation of 4-fluoro-2-((2-fluoro-4-iodophenyl)amino)-5-vinylbenzoic acid

The product (6.2 g) from the previous step, 2-fluoro-4-iodoaniline (8.0 g) and tetrahydrofuran (120 mL) were added into a 500 mL three-necked flask. Subsequently, the system was subjected to nitrogen purging, and cooled to -70°C to -80°C. Lithium bis(trimethylsilyl)amide (1 M, 101 mL) was slowly dropwise added to the system while ensuring that the temperature of the system was not higher than -70°C. After finishing the dropwise addition, the system was slowly allowed to warm to room temperature and maintained at this temperature to react for 30 minutes. Subsequently, hydrochloric acid (1 M) was added to the system to adjust the pH value of the system to be 1-2. The system was extracted with methyl tert-butyl ether, and the organic phases were combined, washed with saturated brine, dried and concentrated. Methyl tert-butyl ether and petroleum ether were slowly added to the concentrate, and the mixture was stirred at room temperature for 30 minutes and filtered to obtain 11.0 g of light brown solid.

### Step 3: preparation of methyl 4-fluoro-2-((2-fluoro-4-iodophenyl)amino)-5-vinylbenzoate

The product (11.0 g) from the previous step and N,N-dimethylformamide (110 mL) were added into a 250 mL three-necked flask, and the system was stirred at room temperature until a clear solution was obtained. Potassium carbonate (5.7 g) was added to the system, and the mixture was heated to 50°C and stirred for 2 hours. The mixture was cooled to room temperature (25°C), and iodomethane (5.8 g) was added to the mixture and allowed to react for 3 hours. The reaction solution was added to an aqueous ammonium chloride solution, the mixture was extracted with ethyl acetate, and the organic phases were combined, washed with saturated brine, dried and concentrated. The concentrate was slurried and purified with methanol to obtain 9.3 g of light brown solid. ¹H NMR (400 MHz, DMSO-*d*₆): δ 9.51 (s, 1H), 8.12 (d, *J =* 8.8 Hz, 1H), 7.76 (dd, *J*₁ = 10.0 Hz, *J*₂ = 1.2 Hz, 1H), 7.57 (d, *J =* 8.4 Hz, 1H), 7.34 (t, *J =* 8.4 Hz, 1H), 6.78-6.67 (m, 2H), 5.76 (d, *J =* 17.6 Hz, 1H), 5.31 (d, *J =* 11.6 Hz, 1H), 3.88 (s, 3H). ESI-MS: [M + H]⁺ = 416.0.

### Step 4: preparation of methyl 4-fluoro-2-((2-fluoro-4-iodophenyl)amino)-5-formylbenzoate

The product (9.3 g) from the previous step and tetrahydrofuran (150 mL) were added into a 500 mL three-necked flask, and the mixture was stirred at room temperature until a clear solution was obtained, and then cooled to 0°C. Potassium osmate dihydrate (413 mg) was added to the system, and the mixture was stirred for 5 minutes. An aqueous solution (150 mL) of sodium periodate (14.4 g) was added to the system, and the mixture was stirred for 5 minutes. Subsequently, 2,4-dimethylpyridine (2.4 g) was added to the system, and the mixture was allowed to react at room temperature for 8 hours. The reaction solution was slowly added to a 20% sodium sulfite solution, the mixture was extracted with ethyl acetate, and the organic phases were combined, dried and concentrated. The concentrate was slurried and purified with ethanol to obtain 5.6 g of light brown solid. ¹H NMR (400 MHz, DMSO-*d*₆): δ 9.95-9.22 (m, 2H), 8.42 (d, *J =* 7.2 Hz, 1H), 7.83 (d, *J =* 9.6 Hz, 1H), 7.64 (d, *J =* 7.6 Hz, 1H), 7.35 (t, *J =* 8.0 Hz, 1H), 6.64 (d, *J =* 13.2 Hz, 1H), 3.91 (s, 3H). ESI-MS: [M + H]⁺ = 418.0.

### Step 5: preparation of methyl (E)-4-fluoro-2-((2-fluoro-4-iodophenyl)amino)-5-((2-p-tolylsulfonylhydrazono)methyl)ben zoate

The product (5.6 g) from the previous step, p-toluenesulfonylhydrazide (2.5 g) and ethanol (120 mL) were added into a 250 mL three-necked flask, and the system was heated to 50°C and allowed to react for 5 hours. The system was cooled to 0°C in an ice bath to precipitate a solid, the mixture was subjected to suction filtration, and the filter cake was washed with ethanol to obtain 7.0 g of gray solid. ¹H NMR (400 MHz, DMSO-*d*₆): δ 11.5 (s, 1H), 9.60 (s, 1H), 8.24 (d, *J =* 8.4 Hz, 1H), 7.95 (s, 1H), 7.77 (dd, *J*₁ = 10.0 Hz, *J*₂ = 1.6 Hz, 1H), 7.73 (d, *J =* 8.4 Hz, 2H), 7.58 (d, *J =* 8.4 Hz, 1H), 7.42 (d, *J =* 8.4 Hz, 2H), 7.32 (t, *J=* 8.4 Hz, 1H), 6.68 (d, *J =* 12.8 Hz, 1H), 3.91 (s, 3H), 2.37 (s, 3H). ESI-MS: [M + H]⁺ *=* 586.0.

### Step 6: preparation of methyl 5-((2-((2,4-dimethoxybenzyl)amino)-3-fluoropyridin-4-yl)methyl)-4-fluoro-2-((2-fluoro-4-iodophenyl)amino)benzoate

(2-((2,4-dimethoxybenzyl)amino)-3-fluoropyridin-4-yl)boronic acid (5.1 g), the product (7.0 g) from the previous step, potassium carbonate (2.8 g) and toluene (300 mL) were added into a 500 mL single-necked flask, and the system was subjected to nitrogen purging. The system was heated to 100°C and allowed to react for 14 hours. After cooling to room temperature, the system was filtered and subjected to rotary evaporation to remove the solvent. Subsequently, the crude product was purified by silica gel column chromatography to obtain 6.0 g of light yellow solid. ¹H NMR (400 MHz, DMSO-*d*₆): δ 9.44 (s, 1H), 7.92 (d, *J =* 8.8 Hz, 1H), 7.74 (dd, *J*₁ = 10.4 Hz, *J*₂ = 1.6 Hz, 1H), 7.65 (d, *J =* 5.2 Hz, 1H), 7.54 (dd, *J*₁ = 8.4 Hz, *J*₂ = 1.2 Hz, 1H), 7.33 (t, *J* = 8.4 Hz, 1H), 7.02 (d, *J =* 8.4 Hz, 1H), 6.84-6.77 (m, 2H), 6.52 (d, *J =* 2.4 Hz, 1H), 6.41 (dd, *J*₁ = 8.4 Hz, *J*₂ = 2.0 Hz, 1H), 6.31 (t, *J =* 4.8 Hz, 1H), 4.43 (d, *J =* 6.0 Hz, 2H). 3.87 (s, 2H), 3.85 (s, 3H), 3.79 (s, 3H), 3.71 (s, 3H). ESI-MS: [M + H]⁺ = 664.1.

### Step 7: preparation of methyl 5-((2-amino-3-fluoropyridin-4-yl)methyl)-4-fluoro-2-((2-fluoro-4-iodophenyl)amino)benzo ate

The product (5.9 g) from the previous step and dichloromethane (118 mL) were added into a 500 mL single-necked flask, and the mixture was stirred at room temperature until a clear solution was obtained. Subsequently, the system was placed in an ice bath, and trifluoroacetic acid (59 mL) was slowly added to the system. After finishing the addition, the system was allowed to warm to room temperature and stirred to react for 1.5 hours. The system was slowly added to a saturated aqueous sodium bicarbonate solution, and the pH value of the system was adjusted to be 8. The mixture was extracted with dichloromethane, and the organic phases were combined and dried with anhydrous sodium sulfate. The mixture was filtered and subjected to rotary evaporation to remove the solvent to obtain 3.9 g of light yellow solid. ESI-MS: [M + H]⁺ = 514.0.

### Step 8: preparation of methyl 4-fluoro-5-((3-fluoro-2-((N-methylsulfamoyl)amino)pyridin-4-yl)methyl)-2-((2-fluoro-4-io dophenyl)amino)benzoate

The product (1.0 g) from the previous step and N,N-dimethylformamide (20 mL) were added into a 100 mL three-necked flask, and stirred until a clear solution was obtained. Subsequently, pyridine (1.6 g) was added to the system, and the mixture was cooled to -15°C. Then, methylsulfamoyl chloride (0.8 g) was dropwise added to the system. After finishing the addition, the system was continuously stirred at -15°C to react for 1 hour. The system was poured into water, the mixture was extracted with ethyl acetate, and the organic phases were combined, dried and concentrated to obtain 1.1 g of brown solid.

### Step 9: preparation of 4-fluoro-5-((3-fluoro-2-((N-methylsulfamoyl)amino)pyridin-4-yl)methyl)-2-((2-fluoro-4-io dophenyl)amino)benzoic acid

The product (1.1 g) from the previous step, tetrahydrofuran (17 mL) and water (9 mL) were sequentially added into a 1 L single-necked flask, and the system was placed in an ice bath and stirred. Lithium hydroxide monohydrate (0.2 g) was slowly added to the system, and after finishing the addition, the mixture was allowed to warm to room temperature and stirred to react for 16 hours. Subsequently, an aqueous hydrochloric acid solution (1 N) was added to the system to adjust the pH value of the system to be 1-2, and the mixture was stirred for 5 minutes. The mixture was extracted with ethyl acetate, and the organic phases were combined, dried and concentrated to obtain 1.0 g of brown solid.

### Step 10: preparation of tert-butyl 2-(4-fluoro-5-((3-fluoro-2-((N-methylsulfamoyl)amino)pyridin-4-yl)methyl)-2-((2-fluoro-4 -iodophenyl)amino)benzoyl)hydrazine-1-carboxylate

The product (1.0 g) from the previous step and N,N-dimethylformamide (20 mL) were added into a 100 mL single-necked flask. Subsequently, 1-hydroxybenzotriazole (0.5 g) and 1-(3-dimethylaminopropyl)-3-ethylcarbodiimide hydrochloride (0.7 g) were added to the system, and the mixture was stirred at room temperature to react for 1 hour. Then, tert-butyl carbazate (0.5 g) and N,N-diisopropylethylamine (0.9 g) were added to the system, and continuously stirred at room temperature to react for 2 hours. The reaction system was slowly added to water, the mixture was extracted with ethyl acetate, and the organic phases were combined, dried and concentrated. The concentrate was purified by silica gel column chromatography to obtain 800 mg of brown solid. ¹H NMR (400 MHz, DMSO-*d*₆): δ 10.28 (s, 1H), 10.25 (s, 1H), 9.69 (s, 1H), 8.91 (s, 1H), 8.00 (d, *J=* 4.8 Hz 1H), 7.76 (s, 1H), 7.69 (dd, *J*₁ = 10.8 Hz, *J*₂ = 1.2 Hz, 1H), 7.51 (d, *J* = 8.4 Hz, 1H), 7.30 (t, *J =* 8.4 Hz, 1H), 6.92-6.88 (m, 3H), 3.94 (s, 2H), 2.53 (d, *J =* 4.8 Hz, 3H), 1.43 (s, 9H).

### Step 11: preparation of 4-fluoro-5-((3-fluoro-2-((N-methylsulfamoyl)amino)pyridin-4-yl)methyl)-2-((2-fluoro-4-io dophenyl)amino)benzohydrazide

The product (800 mg) from the previous step and ethyl acetate (40 mL) were added into a 100 mL single-necked flask, and the system was stirred at room temperature for 5 minutes. Subsequently, a solution of hydrogen chloride in ethyl acetate (4 M, 8 mL) was slowly dropwise added to the system, and continuously stirred at room temperature to react for 4 hours. The reaction system was dropwise added to a saturated aqueous sodium bicarbonate solution, and the system was extracted with ethyl acetate. The organic phases were dried, filtered and concentrated to obtain 640 mg of white solid as a product. ¹H NMR (400 MHz, DMSO-*d*₆): δ 10.38 (s, 1H), 9.93-9.91 (m, 2H), 7.99 (d, *J* = 4.8 Hz 1H), 7.70-7.65 (m, 2H), 7.50-7.47 (m, 1H), 7.30 (t, *J =* 8.4 Hz, 1H), 6.99-6.90 (m, 3H), 4.55 (s, 2H), 3.92 (s, 2H), 2.52-2.51 (m, 3H). ESI-MS: [M + H]⁺ = 607.0.

### Preparation of control compounds

Control compounds TX-1 and TX-2 were prepared according to the reference CN114929669, which were respectively compounds A-13 and A-7 in the reference, with structures as follows:

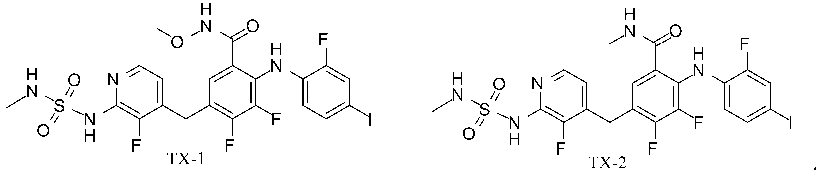

In-vivo therapeutic effect and safety of drugs were results of comprehensive effects of multiple factors, such as inhibitory activity of compounds, various physical and chemical properties, and absorption, metabolism, distribution and excretion. The compounds of the present disclosure showed superior effectiveness and safety, thereby having a good therapeutic prospect.

### Biological Test

### 1. Inhibition activity test on proliferation of pancreatic cancer MIA PaCa-2 cells

Cell proliferation viability was detected by using a CellTiter-3D-Glo reagent assay. An experimental procedure was as follows: MIA-2 cells in a logarithmic growth phase were digested, diluted to a suitable cell density with DMEM complete medium containing 10% FBS, 2.5% HS and 1% PS, seeded into a low-attachment 96-well U-bottom plate, and cultured in 5% CO₂; and test compounds at a series of gradient concentrations were added, and the plate was incubated in a 5% CO² incubator. After finishing the incubation, the cells were lysed and detected by using the CellTiter-3D-Glo reagent. Chemiluminescence values were measured by a microplate reader, and data were fitted by Graphpad Prism software, so as to calculate IC₅₀ values of tested substances. Experimental results can be seen in the table below.

"A" represents inhibitory activity with IC₅₀ < 20 nM, "B" represents inhibitory activity with 20 nM ≤ IC₅₀ < 50 nM, and "C" represents inhibitory activity with IC₅₀ ≥ 50 nM.

| Serial number of compound | IC₅₀ (nM) |
|---|---|
| 3-2 | A |
| 3-8 | A |
| 3-10 | A |
| 4-1 | A |
| 5-1 | A |

The experimental results indicated that the compounds of the present disclosure showed significant cell proliferation inhibitory activity against the pancreatic cancer MIA PaCa-2 cells.

### 2. Pharmacodynamic experiment of subcutaneous xenograft tumor model of pancreatic cancer ASPC-1 cells

### Animal species and number: BALB/c nude mice, 6 mice in each group.

Experimental sample: compound 4-1.

Experimental groups: blank solvent control group and compound 4-1 (0.2 mg/kg, QD × 21 days).

Establishment of animal model: pancreatic cancer ASPC-1 cells in a logarithmic growth phase were cultured in vitro and harvested, and subcutaneously inoculated into the right dorsal side of the Balb/c Nude mice, when tumors grew to about 200-300 mm³, the tumor-bearing mice were randomly divided into groups based on tumor volume, and subsequently the animals in each group were administered with the test compound, wherein the first day of administration was defined as Day 1 of the experiment.

Drug administration route and frequency: oral gavage, once per day, for 21 consecutive days.

General state observation: observation time and frequency: once daily; observation indicators or contents: comprised, but were not limited to, local areas of administration, physical signs, general behaviors, mental states, death and other situations, and other abnormal manifestations of the animals.

Tumor volume calculation: V = 0.5 a × b², wherein a was a long diameter of tumor and b was a short diameter of tumor. A tumor growth inhibition rate TGI (%) was used to evaluate an anti-tumor effect of the compound. TGI (%) *=* [1 - (mean tumor volume of the treatment group at the end of drug administration - mean tumor volume of the treatment group at the start of drug administration) / (mean tumor volume of the control group at the end of drug administration - mean tumor volume of the control group at the start of drug administration)] × 100%.

"+" represents tumor inhibition rate < 60%; "++" represents tumor inhibition rate 60%-100%; and "+++" represents tumor inhibition rate > 100%.

| Compound | TGI (%) |
|---|---|
| 4-1 | +++ |

The experimental results indicated that compound 4-1 showed a significant inhibitory effect on the growth of subcutaneously transplanted tumors of pancreatic cancer ASPC-1 cells in the nude mice. During the drug administration period, the animals in the administration group showed normal diet, body weight and activity, thereby demonstrating good safety.

### 3. Solubility test

Test compound: compound 4-1.

Test procedure: 5 mL of hydrochloric acid solution at pH 1.2 was added into a 25 mL stoppered colorimetric tube, then compound 4-1 was added, the mixture was stirred in a 37°C water bath and filtered through a polyethersulfone membrane, and a concentration of the filtrate was determined by HPLC. Results can be seen in the table below.

"+" represents solubility < 10 µg/mL; "++" represents solubility of 10-30 µg/mL; and "+++" represents solubility > 30 µg/mL.

| Compound | Solubility |
|---|---|
| 4-1 | +++ |

The test results indicated that compound 4-1 had good solubility in the hydrochloric acid solution at pH 1.2.

### 4. Inhibition activity test on proliferation of pancreatic cancer MIA PaCa-2 cells

With reference to Example 1 Biological test, the inhibitory activities of the compounds of the present disclosure on the proliferation of the pancreatic cancer MIA PaCa-2 cells were tested. Experimental results can be seen in the table below. Wherein, "A" represents inhibitory activity with IC₅₀ < 20 nM, "B" represents inhibitory activity with 20 nM ≤ IC₅₀ < 50 nM, and "C" represents inhibitory activity with IC₅₀ ≥ 50 nM.

| Serial number of compound | IC₅₀ (nM) |
|---|---|
| 3-1 | A |
| 3-3 | A |
| 3-9 | A |
| 3-11 | A |
| 3-14 | A |
| 4-2 | A |
| 4-4 | A |
| 4-5 | A |
| 4-6 | A |
| 4-7 | A |

The experimental results indicated that the compounds of the present disclosure had significant cell proliferation inhibitory activity against the pancreatic cancer MIA PaCa-2 cells.

### 5. Inhibitory activity test on proliferation of colorectal cancer SW480 cells

Cell proliferation viability was detected by a CellTiter-3D-Glo reagent assay. An experimental procedure was as follows: SW480 cells in a logarithmic growth phase were digested, diluted to a suitable cell density with DMEM complete medium containing 10% FBS and 1% PS, seeded into a low-attachment 96-well U-bottom plate, and cultured in 5% CO₂; and then test compounds at a series of gradient concentrations were added, and the plate was incubated in a 5% CO₂ incubator. After finishing the incubation, the cells were lysed and detected by using the CellTiter-3D-Glo reagent. Chemiluminescence values were measured by a microplate reader, and data were fitted by Graphpad Prism software, so as to calculate IC₅₀ values of tested substances. Experimental results can be seen in the table below:

| Serial number of compound | IC₅₀ (nM) |
|---|---|
| 3-8 | 0.08 |
| 3-9 | 0.05 |
| 4-1 | 0.03 |
| 4-8 | 0.04 |
| 4-9 | 0.03 |
| 5-1 | 0.08 |
| TX-1 | 0.24 |

The experimental results indicated that the compounds of the present disclosure showed significant cell proliferation inhibitory activity against the colorectal cancer SW480 cells, which was significantly superior to that of the control compound TX-1.

### 6. Inhibitory activity test on proliferation of lung cancer NCI-H358 cells

Cell proliferation viability was detected by a CellTiter-3D-Glo reagent assay. An experimental procedure was as follows: NCI-H358 cells in a logarithmic growth phase were digested and seeded into a low-attachment 96-well U-bottom plate at an appropriate density, and cultured in a cell culture incubator; and media containing test compounds at a series of gradient concentrations were added to experimental wells, while a control medium was added to a control well, and the plate was incubated in a 5% CO₂ incubator. After finishing the incubation, the CellTiter-3D-Glo reagent was added to each well for lysis and detection; and chemiluminescence values were measured by a microplate reader. Data were fitted by Graphpad Prism software, so as to calculate IC₅₀ values of tested substances. Experimental results can be seen in the table below:

| Serial number of compound | IC₅₀ (nM) |
|---|---|
| 3-9 | 0.15 |
| 4-1 | 0.12 |
| 4-8 | 0.13 |
| TX-2 | 2.10 |

The experimental results indicated that the compounds of the present disclosure showed significant cell proliferation inhibitory activity against the lung cancer NCI-H358 cells, which was significantly superior to that of the control compound TX-2.

### 7. Pharmacodynamic experiment of subcutaneous xenograft tumor model of colorectal cancer SW480 cells

### Animal species and number: BALB/c nude mice, 6 mice in each group.

Experimental samples: compound 3-9 and compound 4-1.

Experimental groups: blank solvent control group, compound 3-9 (0.2 mg/kg, QD × 24 days) and compound 4-1 (0.2 mg/kg, QD × 24 days).

Establishment of animal model: SW480 tumor cells in a logarithmic growth phase were cultured in vitro and harvested, and subcutaneously inoculated into the right dorsal side of the Balb/c Nude mice, and when tumors grew to about 180-250 mm³, the tumor-bearing mice were randomly divided into groups. Subsequently, each group of animals were administered with the test compounds, and the first day of drug administration was defined as Day 1 of the experiment.

Drug administration route and frequency: oral gavage, once per day, for 24 consecutive days.

General state observation: the mice were observed once per day, and observation contents comprised, but were not limited to, local areas of administration, physical signs, general behaviors, mental states, death and other situations, and other abnormal manifestations of the animals.

Tumor volume calculation: V = 0.5 a × b², wherein a was a long diameter of tumor and b was a short diameter of tumor. Tumor growth inhibition rates TGI (%) were used to evaluate anti-tumor effects of the compounds. TGI (%) = [1 - (mean tumor volume of the treatment group at the end of drug administration - mean tumor volume of the treatment group at the start of drug administration) / (mean tumor volume of the control group at the end of drug administration - mean tumor volume of the control group at the start of drug administration)] × 100%.

"+" represents tumor inhibition rate < 60%; "++" represents tumor inhibition rate 60%-90%; and +++" represents tumor inhibition rate > 90%.

| Compound | Tumor inhibition rate |
|---|---|
| 3-9 | +++ |
| 4-1 | +++ |

The experiment indicated that the compounds 3-9 and 4-1 showed significant inhibitory effects on the growth of subcutaneously transplanted tumors of colorectal cancer SW480 cells in the nude mice. During the drug administration period, the animals in each administration group showed normal diet, body weight and activity, thereby demonstrating good safety.

## Claims

1. A compound as shown in formula (I), a stereoisomer thereof, a tautomer thereof or a pharmaceutically acceptable salt thereof, 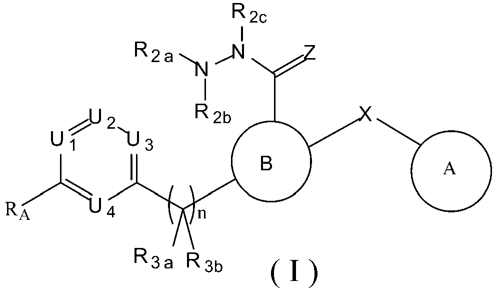
wherein, Z is selected from oxygen or sulfur;
U₁ is selected from nitrogen or CR_{5d}; U₂ is selected from nitrogen or CR₅ₐ; U₃ is selected from nitrogen or CR_{5b}; and U₄ is selected from nitrogen or CR_{5c};
ring B is selected from substituted or unsubstituted aryl or heteroaryl;
R_{A} is selected from -SO₂NRₐR_{b}, -NRₐSO₂NR_{b}R_{c}, -NRₐSO₂R_{b}, -C(O)NRₐR_{b}, -NRₐC(O)NR_{b}R_{c} or -NRₐC(O)R_{b};
each of R₂ₐ, R_{2b} and R_{2c} is independently selected from hydrogen, deuterium, -ORₐ, -SRₐ, C₁₋₆ alkyl, C₂₋₆ alkenyl, C₂₋₆ alkynyl, C₃₋₆ cycloalkyl, 3-10 membered heterocyclyl, C₆₋₁₀ aryl or 5-10 membered heteroaryl, wherein the alkyl, the alkenyl, the alkynyl, the cycloalkyl, the heterocyclyl, the aryl and the heteroaryl are optionally substituted with one or more substituents selected from deuterium, halogen, aldehyde, cyano, amino, hydroxyl, oxo, C₁₋₆ alkyl, C₁₋₆ alkoxy, C₂₋₆ alkenyl, C₂₋₆ alkynyl, C₃₋₆ cycloalkyl, 3-10 membered heterocyclyl, C₆₋₁₀ aryl or 5-10 membered heteroaryl;
each of R₃ₐ, R_{3b}, R₅ₐ, R_{5b}, R_{5c} and R_{5d} is independently selected from hydrogen, deuterium, halogen, aldehyde, cyano, amino, hydroxyl, C₁₋₆ alkyl, C₁₋₆ deuterated alkyl, C₁₋₆ haloalkyl, C₁₋₆ alkoxy, C₁₋₆ alkylthio, C₂₋₆ alkenyl, C₂₋₆ alkynyl or C₃₋₆ cycloalkyl; or two adjacent substituents together form a substituted or unsubstituted 3-10 membered cyclic structure;
X is selected from -O-, -CRₐR_{b} or -NRₐ;
ring A is selected from substituted or unsubstituted aryl or heteroaryl;
each of Rₐ, R_{b} and R_{c} is independently selected from hydrogen, deuterium, C₁₋₆ alkyl, C₁₋₆ deuterated alkyl, C₁₋₆ alkoxy, C₂₋₆ alkenyl, C₂₋₆ deuterated alkenyl, C₂₋₆ alkynyl, C₂₋₆ deuterated alkynyl, C₃₋₆ cycloalkyl, 3-10 membered heterocyclyl, C₆₋₁₀ aryl or 5-10 membered heteroaryl; and
n is 1, 2, 3 or 4.

2. A compound as shown in formula (II), a stereoisomer thereof, a tautomer thereof or a pharmaceutically acceptable salt thereof, 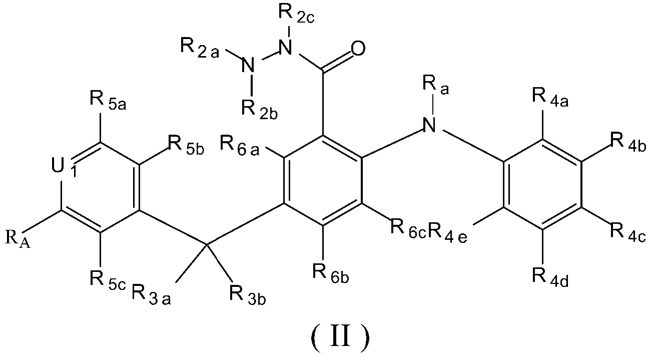
wherein, U₁ is selected from nitrogen or CR_{5d};
R_{A} is selected from -SO₂NRₐR_{b}, -NRₐSO₂NR_{b}R_{c}, -NRₐSO₂R_{b}, -C(O)NRₐR_{b}, -NRₐC(O)NR_{b}R_{c} or -NRₐC(O)R_{b};
each of Rₐ, R_{b} and R_{c} is independently selected from hydrogen, deuterium, C₁₋₆ alkyl, C₁₋₆ deuterated alkyl, C₁₋₆ alkoxy, C₂₋₆ alkenyl, C₂₋₆ deuterated alkenyl, C₂₋₆ alkynyl, C₂₋₆ deuterated alkynyl, C₃₋₆ cycloalkyl, 3-10 membered heterocyclyl, C₆₋₁₀ aryl or 5-10 membered heteroaryl; and
each of R₂ₐ, R_{2b} and R_{2c} is independently selected from hydrogen, deuterium, -ORₐ, -SRₐ, C₁₋₆ alkyl, C₂₋₆ alkenyl, C₂₋₆ alkynyl, C₃₋₆ cycloalkyl, 3-10 membered heterocyclyl, C₆₋₁₀ aryl or 5-10 membered heteroaryl, wherein the alkyl, the alkenyl, the alkynyl, the cycloalkyl, the heterocyclyl, the aryl and the heteroaryl are optionally substituted with one or more substituents selected from deuterium, halogen, aldehyde, cyano, amino, hydroxyl, oxo, C₁₋₆ alkyl, C₁₋₆ alkoxy, C₂₋₆ alkenyl, C₂₋₆ alkynyl, C₃₋₆ cycloalkyl, 3-10 membered heterocyclyl, C₆₋₁₀ aryl or 5-10 membered heteroaryl; and
each of R₃ₐ, R_{3b}, R₄ₐ, R4b, R_{4c}, R4d, R₄ₑ, R₅ₐ, R_{5b}, R_{5c}, R_{5d}, R₆ₐ, R6b and R_{6c} is independently selected from hydrogen, deuterium, halogen, aldehyde, cyano, amino, hydroxyl, C₁₋₆ alkyl, C₁₋₆ deuterated alkyl, C₁₋₆ haloalkyl, C₁₋₆ alkoxy, C₁₋₆ alkylthio, C₂₋₆ alkenyl, C₂₋₆ alkynyl or C₃₋₆ cycloalkyl; or two adjacent substituents together form a substituted or unsubstituted 3-10 membered cyclic structure.

3. A compound as shown in formula (III), a stereoisomer thereof, a tautomer thereof or a pharmaceutically acceptable salt thereof, 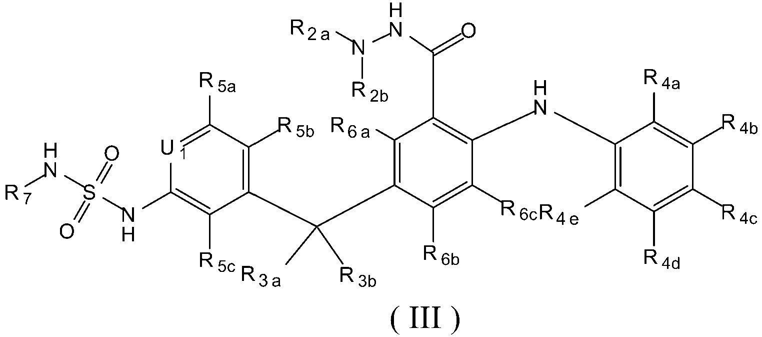
wherein, U₁ is selected from nitrogen or CR_{5d};
each of R₂ₐ and R_{2b} is independently selected from hydrogen, deuterium, -ORₐ, -SRₐ, C₁₋₆ alkyl, C₂₋₆ alkenyl, C₂₋₆ alkynyl, C₃₋₆ cycloalkyl, 3-10 membered heterocyclyl, C₆₋₁₀ aryl or 5-10 membered heteroaryl, wherein the alkyl, the alkenyl, the alkynyl, the cycloalkyl, the heterocyclyl, the aryl and the heteroaryl are optionally substituted with one or more substituents selected from deuterium, halogen, aldehyde, cyano, amino, hydroxyl, oxo, C₁₋₆ alkyl, C₁₋₆ alkoxy, C₂₋₆ alkenyl, C₂₋₆ alkynyl, C₃₋₆ cycloalkyl, 3-10 membered heterocyclyl, C₆₋₁₀ aryl or 5-10 membered heteroaryl;
Rₐ is selected from hydrogen, deuterium, C₁₋₆ alkyl, C₁₋₆ deuterated alkyl, C₂₋₆ alkenyl, C₂₋₆ deuterated alkenyl, C₂₋₆ alkynyl, C₂₋₆ deuterated alkynyl or C₃₋₆ cycloalkyl;
each of R₃ₐ and R_{3b} is independently selected from hydrogen, deuterium, halogen or C₁₋₆ alkyl;
each of R₄ₐ, R4b, R_{4c}, R_{4d}, R₄ₑ, R₅ₐ, R_{5b}, R_{5c}, R₅ₐ, R₆ₐ, R_{6b} and R_{6c} is independently selected from hydrogen, deuterium, halogen, aldehyde, cyano, amino, hydroxyl, C₁₋₆ alkyl, C₁₋₆ deuterated alkyl, C₁₋₆ haloalkyl, C₁₋₆ alkoxy, C₁₋₆ alkylthio, C₂₋₆ alkenyl, C₂₋₆ alkynyl or C₃₋₆ cycloalkyl; or two adjacent substituents together form a substituted or unsubstituted 3-10 membered cyclic structure; and
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl or C₂₋₆ alkynyl.

4. A compound as follows, a stereoisomer thereof, a tautomer thereof or a pharmaceutically acceptable salt thereof,

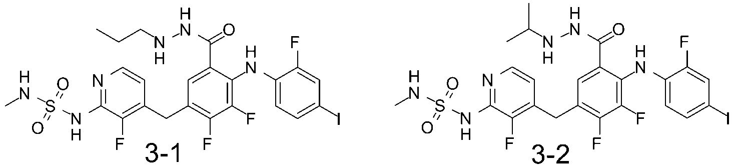

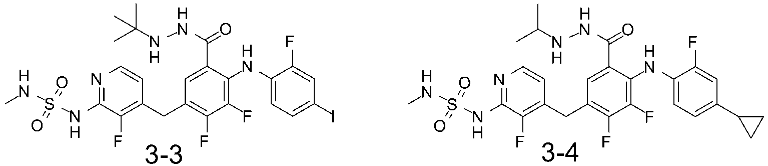

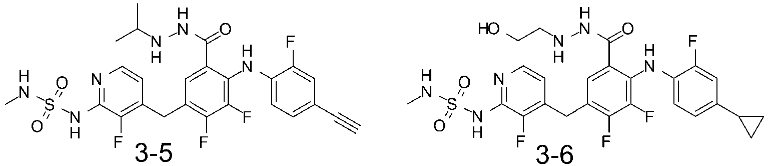

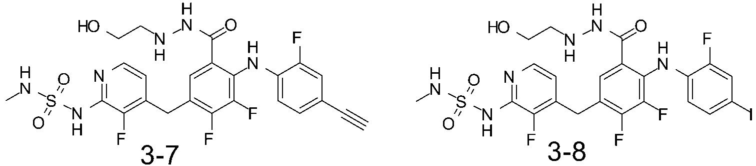

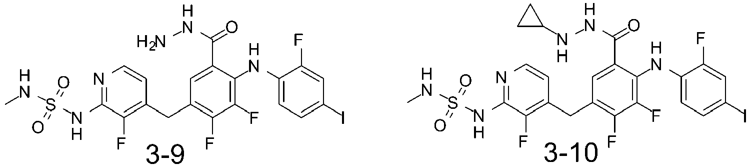

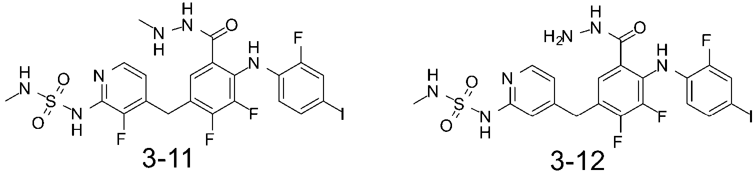

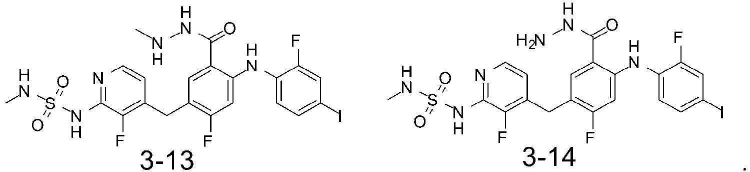

5. A compound as shown in formula (IV- I), a stereoisomer thereof, a tautomer thereof or a pharmaceutically acceptable salt thereof, 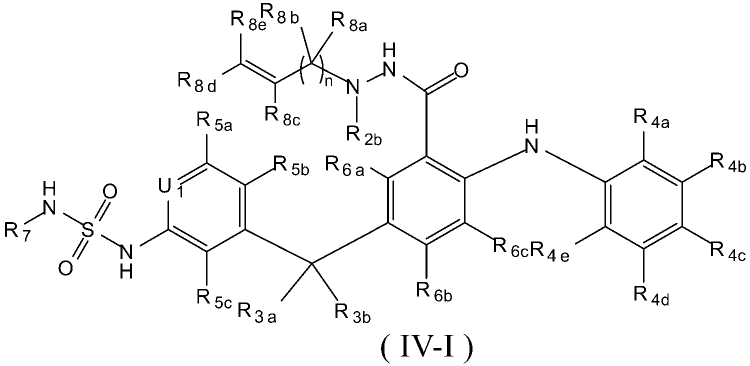
wherein, U₁ is selected from nitrogen or CR_{5d};
R_{2b}, R₃ₐ, R_{3b} are selected from hydrogen, deuterium, halogen, C₁₋₆ alkyl, C₁₋₆ deuterated alkyl or C₁₋₆ haloalkyl;
each of R₄ₐ, R4b, R_{4c}, R_{4d}, R₄ₑ, R₅ₐ, R_{5b}, R_{5c}, R₅ₐ, R₆ₐ, R6b and R_{6c} is independently selected from hydrogen, deuterium, halogen, aldehyde, cyano, amino, hydroxyl, C₁₋₆ alkyl, C₁₋₆ deuterated alkyl, C₁₋₆ haloalkyl, C₁₋₆ alkoxy, C₁₋₆ alkylthio, C₂₋₆ alkenyl, C₂₋₆ alkynyl or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl or C₂₋₆ alkynyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl, C₁₋₆ haloalkyl or C₃₋₆ cycloalkyl; and
n is 1, 2, 3 or 4.

6. A compound as shown in formula (IV- II), a stereoisomer thereof, a tautomer thereof or a pharmaceutically acceptable salt thereof, 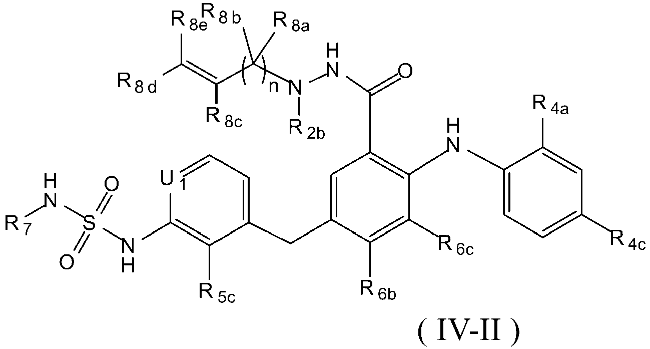
wherein, U₁ is selected from nitrogen or CH;
R_{2b} is selected from hydrogen, deuterium or C₁₋₆ alkyl;
each of R₄ₐ and R_{4c} is independently selected from hydrogen, deuterium, halogen, aldehyde, hydroxyl, C₁₋₆ alkyl, C₁₋₆ alkoxy, C₂₋₆ alkenyl, C₂₋₆ alkynyl or C₃₋₆ cycloalkyl;
each of R_{5c}, R_{6b} and R_{6c} is independently selected from hydrogen, deuterium, halogen or C₁₋₆ alkyl;
R₇ is selected from hydrogen, C₁₋₆ alkyl, C₁₋₆ deuterated alkyl or C₃₋₆ cycloalkyl;
each of R₈ₐ, R_{8b}, R_{8c}, R_{8d} and R₈ₑ is independently selected from hydrogen, deuterium, halogen, C₁₋₆ alkyl, C₁₋₆ deuterated alkyl or C₁₋₆ haloalkyl; and
n is 1, 2, 3 or 4.

7. A compound as shown in formula (IV-III), a stereoisomer thereof, a tautomer thereof or a pharmaceutically acceptable salt thereof, 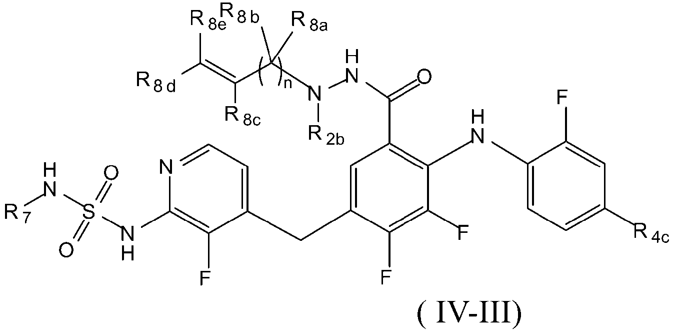
wherein, R_{2b} is selected from hydrogen or C₁₋₆ alkyl;
R_{4c} is selected from halogen, aldehyde, C₁₋₆ alkyl, C₁₋₆ alkoxy, C₂₋₆ alkenyl, C₂₋₆ alkynyl or C₃₋₆ cycloalkyl;
R₇ is selected from C₁₋₆ alkyl, C₁₋₆ deuterated alkyl or C₃₋₆ cycloalkyl;
each of R₈ₐ, R_{8b}, R_{8c}, R_{8d} and R₈ₑ is independently selected from hydrogen, deuterium or C₁₋₆ alkyl; and
n is 1, 2, 3 or 4.

8. A compound as follows, a stereoisomer thereof, a tautomer thereof or a pharmaceutically acceptable salt thereof,

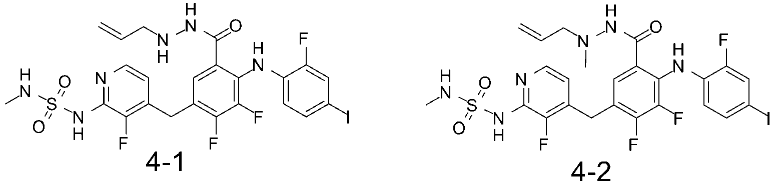

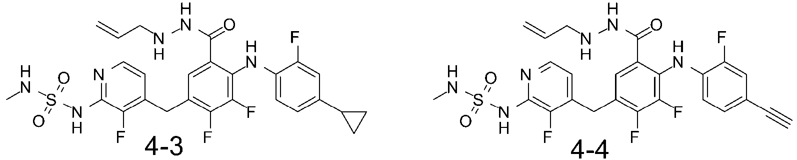

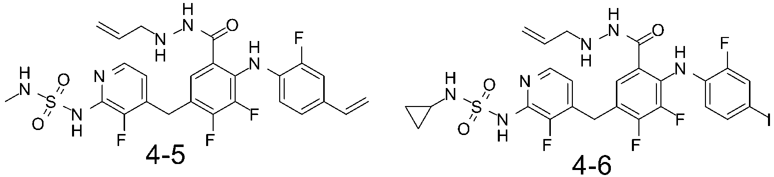

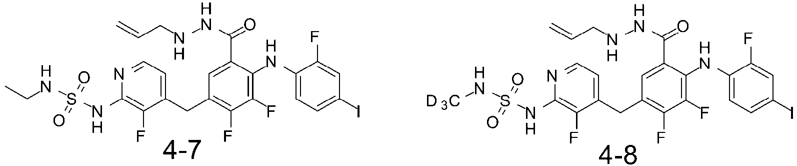

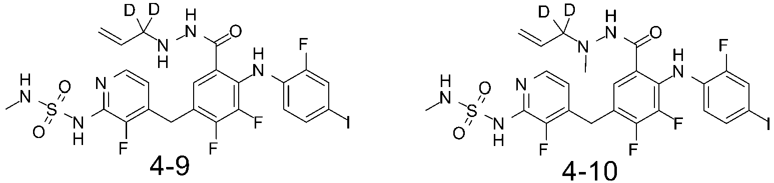

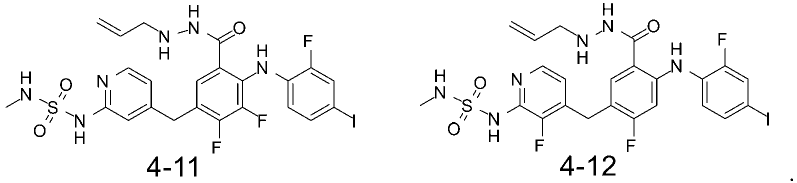

9. A compound as shown in formula (V), a stereoisomer thereof, a tautomer thereof or a pharmaceutically acceptable salt thereof, 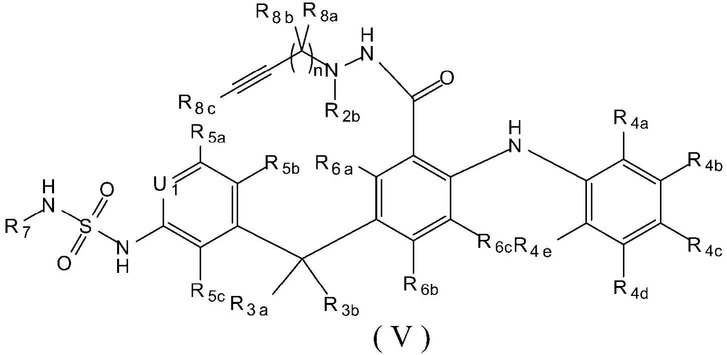
wherein, U₁ is selected from nitrogen or CR_{5d};
R_{2b}, R₃ₐ, R_{3b} are selected from hydrogen, deuterium, halogen, C₁₋₆ alkyl, C₁₋₆ deuterated alkyl or C₁₋₆ haloalkyl;
each of R₄ₐ, R4b, R_{4c}, R_{4d}, R₄ₑ, R₅ₐ, R_{5b}, R_{8c}, R₅ₐ, R₆ₐ, R_{6b} and R_{6c} is independently selected from hydrogen, deuterium, halogen, aldehyde, cyano, amino, hydroxyl, C₁₋₆ alkyl, C₁₋₆ deuterated alkyl, C₁₋₆ haloalkyl, C₁₋₆ alkoxy, C₁₋₆ alkylthio, C₂₋₆ alkenyl, C₂₋₆ alkynyl or C₃₋₆ cycloalkyl; or two adjacent substituents together form a substituted or unsubstituted 3-10 membered cyclic structure;
R₇ is selected from hydrogen, C₁₋₆ alkyl, C₁₋₆ deuterated alkyl, C₁₋₆ haloalkyl, C₁₋₆ alkoxy, C₃₋₆ cycloalkyl, C₂₋₆ alkenyl or C₂₋₆ alkynyl;
each of R₈ₐ, R_{8b} and R_{8c} is independently selected from hydrogen, deuterium, halogen, C₁₋₆ alkyl, C₁₋₆ deuterated alkyl, C₁₋₆ haloalkyl or C₃₋₆ cycloalkyl; and
n is 1, 2, 3 or 4.

10. A compound as follows, a stereoisomer thereof, a tautomer thereof or a pharmaceutically acceptable salt thereof,

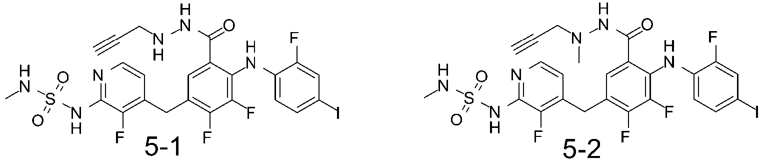

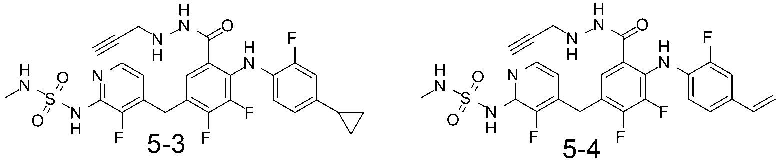

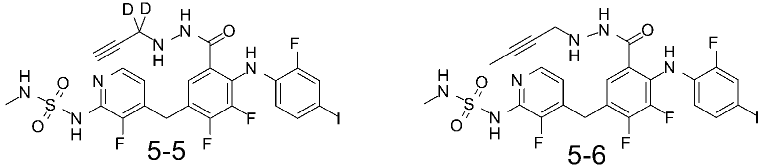

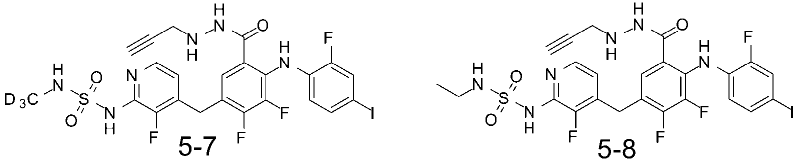

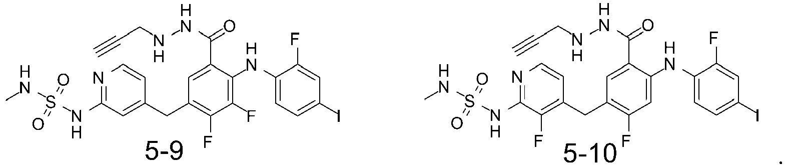

11. A pharmaceutical composition, comprising a therapeutically effective amount of the compound, the stereoisomer thereof, the tautomer thereof or the pharmaceutically acceptable salt thereof according to any one of claims 1 to 10, and a pharmaceutically acceptable carrier.

12. Use of the compound, the stereoisomer thereof, the tautomer thereof or the pharmaceutically acceptable salt thereof according to any one of claims 1 to 10, or the pharmaceutical composition according to claim 11 in the manufacture of a medicament for the prevention and/or treatment of a tumor.

13. Use of the compound, the stereoisomer thereof, the tautomer thereof or the pharmaceutically acceptable salt thereof according to any one of claims 1 to 10, or the pharmaceutical composition according to claim 11 in the manufacture of a medicament for the prevention and/or treatment of a MAPK pathway-related disease, wherein the disease particularly refers to a tumor.

14. The use according to claim 12 or 13, wherein the tumor comprises kidney cancer, liver cancer, breast cancer, pancreatic cancer, prostate cancer, melanoma, leukemia, malignant lymphoma, ovarian cancer, head and neck cancer, lung cancer, colorectal cancer, bladder cancer, uterine cancer, and the like.
